# EUROPEAN PATENT APPLICATION

(11) **EP 1 002 873 A2**
(43) Date of publication of application: **24.05.2000**
(21) Application number: 99305099.6
(22) Date of filing: 29.06.1999
(51) Int. Cl.: C12Q 1/26, C12Q 1/28

(54) **Novel assay systems for vanadium bromoperoxidase**

(30) Priority: 29.06.1998 US 91033 P; 03.06.1999 US 325521
(71) Applicant: Ortho-Clinical Diagnostics, Inc., Rochester, NY 14626-5101 (US)
(72) Inventor: Detty, Michael R., Rochester, NY 14617-2117 (US); Friedman, Alan E., Rochester, NY 14617 (US); Groulx, Sarah F., Ontario, NY 14519 (US); Snoke, Roy E., Webster, NY 14580 (US); Weber II, Wayne W., Mendon, NY 14472 (US)
(74) Representative: Mercer, Christopher Paul

(57) **Abstract**

Analytical systems and assays are disclosed which can be used to sensitively and rapidly detect a wide variety analytes. The assays are carried out using haloperoxidase, halide, peroxide and a signal-generating compound which upon halogenation provides a detectable change in spectral absorption.

## Description

The present invention relates to analytical systems and methods for detecting analytes. More particularly, the present invention relates to analytical systems and methods using vanadium bromoperoxidase and specific signal-generating compounds.

### Background of the Invention

There is a continuing need in medical practice and research for rapid and accurate determinations of chemical and biological substances that are present in various fluids, such as biological fluids. For example, the presence of glucose, urea, triglycerides, bilirubin, cholesterol, lipase, lactate, proteins, hormones, drugs, viruses, microorganisms, narcotics, nucleic acids, oligonucleotides, steroids, and the like, must be determined rapidly and accurately for effective research, diagnosis and treatment.

Many analytical assays are available. However, reagent and/or signal stability is often a problem; compromising the accuracy and precision of the analytical measurement and limiting the detection sensitivity.

It is to be understood that the terms analytical methods, assays and systems encompass solution-based analytical methods and compositions, that is, systems wherein reagents are in liquid form prior to contact with a sample comprising an analyte, and dry systems wherein reagents are in dry form prior to contact with a sample comprising an analyte. Included are systems wherein the analyte to be determined is capable of undergoing chemical reaction with one or more reagents to produce a product that is capable of being qualitatively and/or quantitatively detected. Also included are systems that involve binding reactions, wherein an analyte to be measured (which is not typically altered chemically, although it can be) bonds noncovalently to a specific binding partner in competition with labeled-analyte in competitive assays, or wherein a labeled-receptor binds to the analyte or another specific binding partner which itself is bound to the analyte, or which in turn is bound to a receptor which itself is bound to the analyte, and so on, in what has become known in the art as sandwich or immunometric assays. In general, one of the receptors specific to the analyte is immobilized or is capable of being immobilized. It is understood that a labeled or unlabeled receptor that is bound to an analyte or that is bound to another receptor that is bound to the analyte and so on, which analyte in turn is bound to an immbobilized or immobilizable receptor will thus be bound to the immobilized or immobilizable receptor by way of the complex formed therein. The term labeled-analyte shall be understood to include labeled analogs and derivatives of the analyte.

Binding assays that involve a monoclonal or polyclonal antibody, or antibody fragment, such as an Fab fragment, as a binding partner are often referred to as immunoassays. Binding systems or assays as described hereinabove, encompass immunoassays.

Specific binding partners that relate to the present invention include but are not limited to: antibody and anti-antibody, antibody and hapten, sugar and lectin, nucleic acid oligonucleotide probe and target nucleic acid sequence, enzyme and cofactor, avidin and biotin, and strepavidin and biotin. Typical analytes that involve binding reactions include but are not limited to: steroids, drugs, oligonucleotides, proteins, sugars, saccharides, oligo- and polysaccharides.

The detection of specific nucleic acid sequences using labeled-oligonucleotides in sandwich assays is well known. Labeled-oligonucleotides for use in assays based on nucleic acid amplification technology such as the polymerase chain reaction (PCR) enjoy particular utility. PCR and the application of labeled-oligonucleotides in PCR is described in numerous publications, for example, U.S. Patent No. 5,229,297 to Schnipelsky et al., U.S. Patent No. 5,328,825 to Warren et al., and U.S. Patent No. 5,387,510 to Wu.

Solution-based analytical systems are extensively employed. A widely accepted alternative to a solution-based analytical system is a dry analytical element. Dry analytical elements and their use in analytical assays are described in numerous publications, including U.S. Patent No. 4,258,001 to Pierce et al., U.S. Patent No. 4,670,381 to Frickey et al., WO 82/2601 (published August 5, 1982), European Patent Application No. 051 183 (published May 12, 1982) and European Patent Application No. 066 648 (published December 15, 1982).

Improved dry analytical elements and their use in binding assays are described in EP-A-0 587 222.
in which enzyme labels are utilized for detection. Horseradish peroxidase is the preferred enzyme label disclosed in the application of Belly et al. Such elements allow for the detection of analytes present in very low concentrations using a particular washing technique to separate free reactants from bound reactants.

The enzyme horseradish peroxidase (HRP) has been utilized as a means for amplifying a target-related signal (wherein HRP acts to produce many molecules of signal-generating product in unit time per molecule of target analyte present in a sample of interest) in rate, fixed or endpoint-type assays. HRP catalyzes oxidations with hydrogen peroxide in which the oxidized form of the enzyme removes electrons from a compound. If the compound is a triarylimidazole leuco dye, for example those disclosed in U.S. Patent No. 4,089,747 to Bruschi, oxidation of the leuco form produces a dye as the signal-generating product. The time rate of change of signal intensity or the signal intensity measured at some fixed or endpoint in time is related to the concentration of the analyte being evaluated.

HRP has several limitations. HRP works best in an aqueous environment near neutral pH. It requires stabilizing agents and enhancers as disclosed in U.S. Patent No. 4,828,983 to McClune, U.S. Patent No. 5,372,931 and U.S. Patent No. 5,372,932 to Friedman et al. While low levels of organic cosolvents are tolerated, the enzyme readily denatures as water is replaced with organic solvents.

In binding assays carried out with peroxidase as label, the stability of the peroxidase is highly important since any change in its concentration critically affects assay sensitivity. This is particularly acute in dry analytical elements. In the assays described in U.S. Patent No. 5,372,932 to Friedman et al., 4'-hydroxy or 4'-alkoxyarylacetamides are used as agents to enhance the stability of the enzyme or enzyme label in a dry analytical element.

HRP is a heme-containing enzyme. Stability of the enzyme is a function of a wide range of environmental factors including temperature, humidity, metals, solvents, ionic strength, organic and inorganic oxidants, reductants, reactive oxygen species such as, superoxide, hydroxyl radical, singlet oxygen, and peroxide; evidently related to the susceptibility of the heme moiety.

The use of vanadyl compounds is described in U.S. Patent No. 5,516,645 to Daniel et al. and diaryl tellurides in EP-A-0 747 704 to reduce "first slide bias". First slide bias is an instability of the peroxidase label in the uppermost slide element, which instability is due to reactive oxygen species that act on the heme moiety in heme-containing peroxidases to cause inactivation of the enzyme. This source of peroxidase instability is not the cause of storage instability that is alleviated by the stabilizers disclosed in the aforementioned '932 patent.

Although the agents disclosed in all the above patents have resulted in improved stability, it has been observed that the stability of the peroxidase label is still less than desirable.

A catalytic system is required with hydrogen peroxide oxidation of leuco dyes, such as di and triarylimidazoles, because the rate of oxidation with H₂O₂ is extremely slow in the absence of catalyst.

Hydrogen peroxide is a powerful oxidant from a thermodynamic perspective and an environmentally friendly oxidant yielding oxygen and water upon decomposition. Many reactions of H202, which are favored thermodynamically, are limited by the kinetics of reaction. Two such reactions are the oxidations of bromide to bromine/hypobromous acid and chloride to chlorine/ hypochlorous acid with H202, which are slow at ambient temperature and neutral pH [Mohammed, A.; Liebhafsky, H. A. *J. Am. Chem. Soc.* 1934, 56, 1680-1685]. In nature, halide oxidations with peroxide are catalyzed by haloperoxidase enzymes [Butler, A.; Carrano, C. *J. Coord. Chem. Rev.* 1991, 109, 61-105; Wever, R.; Kreenn, M. B. E. *Vanadium in Biological Systems;* Chasteen, N. D., Ed., Kluwer Academic Publishers: Dordrecht, The Netherlands, 1990; pp 81-97; Butler, A.; Walker, J. V. *Chem. Rev.* 1993, 93, 1937-1944; Butler, A. *Bioinorganic Catalysis;* Reedijk, J., Ed.; Marcel Dekker: New York, 1992; pp 425-445]. Chloroperoxidase [Dexter, A. F.; Lakner, F. J.; Campbell, R. A.; Hager, L. P. *J. Am. Chem. Soc.* 1995, 117, 6412-6413; Allain, E. J.; Hager, L. P.; Deng, L.; Jacobsen, E. N. *J. Am. Chem. Soc.* 1993, 115, 4415-4416] and bromoperoxidase [Butler, A.; Carrano, C. *J. Coord. Chem. Rev.* 1991, 109, 61-105; Wever, R.; Kreenn, M. B. E. *Vanadium in Biological Systems;* Chasteen, N. D., Ed., Kluwer Academic Publishers: Dordrecht, The Netherlands, 1990; pp 81-97; Butler, A.; Walker, J. V. *Chem. Rev.* 1993, 93, 1937-1944; Butler, A. *Bioinorganic Catalysis;* Reedijk, J., Ed.; Marcel Dekker: New York, 1992; pp 425-445] enzymes and model systems that mimic their activity [Collman, J. P.; Lee, V. J.; Kellen-Yuen, C. J.; Zhang, X.; Ibers, J. A.; Brauman, J. I. *J. Am. Chem.* Soc. 1995, 117, 692-703; Palucki, M.; Pospisil, P. J.; Zhang, W.; Jacobsen, E. N. *J. Am. Chem. Soc.* 1994, 116, 9333-9334; Jacobsen, E. N.; Zhang, W.; Muci, A. R.; Ecker, J. R.; Deng, L. *J. Am. Chem. Soc.* 1991, 113, 7063-7064; Lee, N. H.; Jacobsen, E. N. *Tetrahedron Lett.* 1991, 32, 6533-6536; Andersson, M.; Conte, V.; Di Furia, F.; Moro, S. *Tetrahedron Lett.* 1995, 36, 2675-2678; Clague, M. J.; Butler, A. *J. Am. Chem. Soc.* 1995, 117, 3475-3484; Meister, G. E.; Butler, A. *Inorg. Chem.* 1994, 33, 3269-3275; Colpas, G. J.; Hamstra, B. J.; Kampf, J. W.; Pecoraro, V. L. *J. Am. Chem. Soc.* 1994, 116, 3627-3628; Reynolds, M. S.; Morcandi, S. J.; Raebiger, J. W.; Miliccin, S. P.; Smith, S. P. E. *Inorg. Chem.* 1994, 33, 4977-4984; Espenson, J. H.; Pestovsky, O.; Huston, P., Staudt, S. *J. Am. Chem. Soc.* 1994, 116, 2869-2877; Ma, R.; Bakac, A.; Espenson, J. H. *Inorg. Chem.* 1992, 31, 1925-1930; de la Rosa, R. I.; Clague, M. J.; Butler, A. *J. Am. Chem. Soc.* 1992, 114, 760-761] utilize a transition metal (heme-bound iron for chloroperoxidase, non-heme vanadium for bromoperoxidase) [Butler, A.; Walker, J. V. *Chem. Rev.* 1993, 93, 1937-1944] to activate H₂O₂ for the oxidation of halide to halogen or hypohalous acid. The production of halogen and/or hypohalous acid with the haloperoxidase enzymes could be utilized in an amplification system if appropriate signal-generating compounds were available.

Butler and Walker have described a family of non-heme vanadium bromoperoxidases extracted from aquatic and marine algae and some from terrestrial lichens and fungi that, in the presence of hydrogen peroxide and bromide anion, catalytically produce activated bromine species which are potent oxidants (Butler et al., *Chem. Revs.,* 93, pp. 1937-1944, 1993 and references cited therein.

Many analytical systems depend on the generation of an optical absorption change that is related to the amount of an analyte in a sample of interest. For example, the triarylimidazole leuco dyes described in U.S. Patent No. 4,089,747 are capable of being oxidized in the presence of horseradish peroxidase and peroxide, as stated. Upon oxidation, a triarylimidazole leuco dye having insignificant absorption in the visible portion of the electromagnetic spectrum, is converted to a dye having significant absorption in the visible portion of the spectrum. These and other compounds of the prior art that react with peroxide and peroxidase to produce detectable product have found wide use in analytical chemistry.

Unfortunately, the aforementioned leuco dyes are not stable. In the absence of peroxidase and peroxide leuco dyes are capable of being oxidized by oxygen present in air resulting in formation of dye. This background "color-up" worsens in time and eventually will render an assay system useless.

Additionally, the resulting dye formed upon oxidation of precursor leuco dye is not stable; it is light sensitive and fades over time.

Use of haloperoxidase, particularly non-heme vanadium bromoperoxidase, is not widely known in analytical chemistry. The application of non-heme vanadium bromoperoxidase in chemiluminesence analytical systems is described in EP-A-0 892 855 and EP-A-0 848 819.

A colorimetric assay for vanadium bromperoxidase is discussed in de Boer et al., *Biotechnology and Bioengineering,* v30, pp 607-610, 1987.

Bromination of the pH indicator dye, phenol red, to produce bromophenol blue is described. Colorimetric detection of vanadium bromoperoxidase, in the case of such compounds, relies on the difference in pKa produced upon bromination. A pH indicator dye is generally useful only at a pH permitting nearly complete proton dissociation or association.

A method for detecting haloperoxidase activity relying on the loss of absorption at 290 nm upon halogenation of monochlorodimedone (MCD) by haloperoxidase is described in Butler et al., *Chem. Rev.,* v93, pp1937-1944 (1993). Due to instability of MCD at neutral pH and competing side reactions, as described in the aforementioned Butler et al. reference, this system is not desirable for analytical use.

The change in spectral absorption upon haloperoxidase catalyzed halogenation of phenol red or MCD at a given wavelength is not linear as a function of time or analyte concentration due to competing side reactions and formation of multiple halogenated species having different spectral absorption characteristics.

Although the analytical systems and methods disclosed in the prior art show enhanced assay sensitivity, specificity, and stability there is still a need for further improvements in this field. For example, there is still a need for providing simpler analytical systems which do not require the use of stabilizing agents as described in U.S. Patent No. 5,372,932 to Friedman et al. or enhancers as in U.S. Patent No. 4,828,983 to McClune. Furthermore, there still is a need to provide analytical assays having increased signal measurement sensitivity, stability, and versatility than the known prior art systems.

### Summary of the Invention

The problems identified above have been overcome by employing a haloperoxidase and specific signal-generating compounds, which compounds upon halogenation or halogenation and subsequent nucleophilic addition, produce sensitive and stable spectroscopic absorption signals.

The present invention can be used in solution-based analytical systems and dry reagent embodiments.

Signal-generating compounds useful in the practice of this invention have structural formulas represented in DW1 through DW4 below.

*Compounds designated DW1 have the structure:* 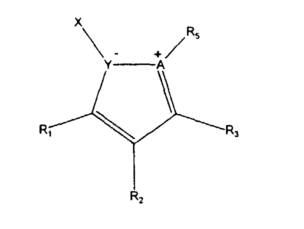 wherein Y is Te or Se; A is N, O, or S; X is Cl, Br, I, F, CN, SCN or CF₃CO₂ ; R₁, R₂ and R₃ are independently H, substituted or unsubstituted straight chain or branched alkyl, oxyalkyl, thioalkyl, aminoalkyl of 1 to 6 carbon atoms, substituted or unsubstituted alkenyl of 2 to 6 carbon atoms, substituted or unsubstituted aryl or heteroaryl of 6 to 14 carbon atoms, Cl, Br, I, or F, or R₁ and R₂ together form a substituted, or unsubstituted benzene ring or naphthalene ring; R₅ is an electron pair when when A is S, or O; R₅ is H, substituted or unsubstituted straight chain or branched alkyl, oxyalkyl, thioalkyl, aminoalkyl of 1 to 6 carbon atoms, or substituted or unsubstituted alkenyl of 2 to 6 carbon atoms when A is N.

*Compounds designated DW1nuc have the structure:* 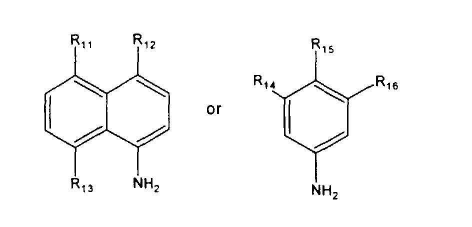 wherein R₁₁, R₁₂, R₁₃ are independently H, OH, or NMe₂ and R₁₄, R₁₅, R₁₆ are independently H, OH, NMe₂ or halogen.

*Compounds designated DW2 have the following structural formula:* 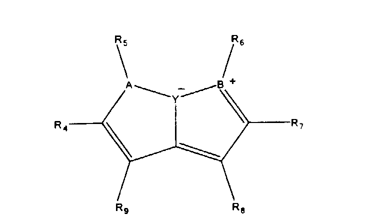 wherein Y, A, and R₅ are as defined for DW1; B is N, O, or S; R₄ and R₇ are independently H, amine, hydroxy, substituted or unsubstituted straight chain or branched alkyl, oxyalkyl, thioalkyl, aminoalkyl of 1 to 6 carbon atoms, substituted or unsubstituted alkenyl of 2 to 6 carbon atoms, substituted or unsubstituted heterocyclic ring of 5 or 6 atoms, substituted or unsubstituted aryl or heteroaryl of 6 to 14 carbon atoms; R₈ and R₉ are independently H, amine, hydroxy, Cl, Br, I, F, substituted or unsubstituted straight chain or branched alkyl, oxyalkyl, thioalkyl, aminoalkyl of 1 to 6 carbon atoms, substituted or unsubstituted alkenyl of 2 to 6 carbon atoms, substituted or unsubstituted heterocyclic ring of 5 or 6 atoms, substituted or unsubstituted aryl or heteroaryl of 6 to 14 carbon atoms; R₄ and R₉ together form a substituted or unsubstituted benzene or naphthalene ring; R₇ and R₈ together form a substituted or unsubstituted benzene or naphthalene ring; R₆ is an electron pair when when B is S, or O; R₆ is H, substituted or unsubstituted straight chain or branched alkyl, oxyalkyl, thioalkyl, aminoalkyl of 1 to 6 carbon atoms, or substituted or unsubstituted alkenyl of 2 to 6 carbon atoms when B is N.

*Compounds designated DW3 have the following structural formula:* 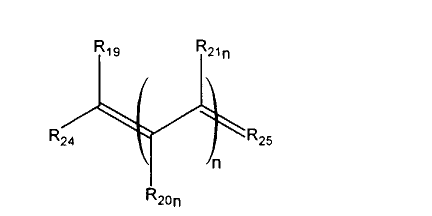 n is an integer from 0 to 8, R19, R20n and R21n are independently hydrogen, halogen, cyano, amino, substituted or unsubstituted alkyl, thioalkyl, oxyalkyl, aminoalkyl, amidoalkyl or N-substituted amidoalkyl of 1 to 6 carbon atoms, substituted or unsubstituted aryl, thioaryl or oxyaryl, R24 is 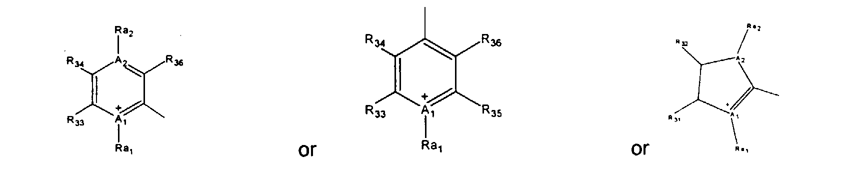 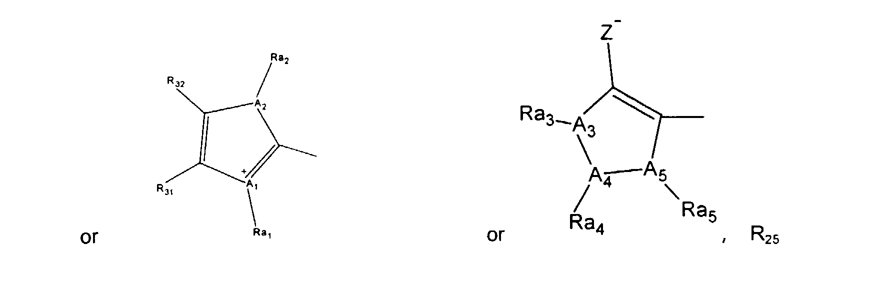 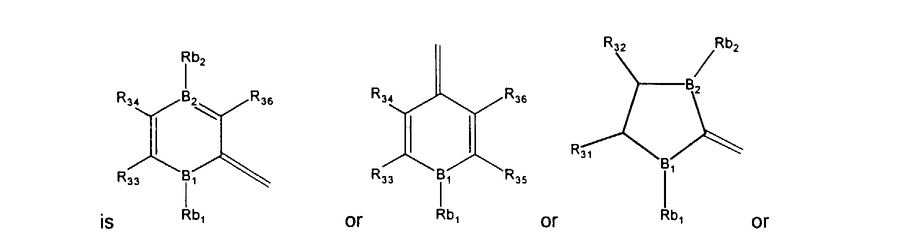 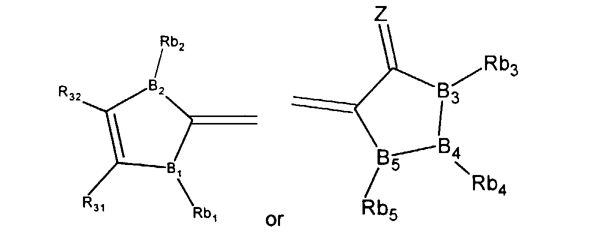 A₁,A₂,B₁ and B₂ are independently Se,Te,C,N,S,O; Ra₁, Ra₂, Rb₁ and Rb₂ are independently H,substituted or unsubstituted straight chain or branched alkyl, oxyalkyl, thioalkyl, aminoalkyl of 1 to 6 carbon atoms, or substituted or unsubstituted alkenyl of 2 to 6 carbon atoms when A₁,A₂,B₁ or B₂ is C or N, and an electron pair when when A₁,A₂,B₁ or B₂ is Se, Te, O, or S; R₃₁ and R₃₂ are independently H, amine, hydroxy, halide, acetyl, acetylamino, substituted or unsubstituted straight chain or branched alkyl, thioalkyl, aminoalkyl, oxyalkyl of 1 to 6 carbon atoms, substituted or unsubstituted alkenyl of 2 to 6 carbon atoms, substituted or unsubstituted aryl or heteroaryl of 6 to 14 atoms, or R₃₁ and R₃₂ together form a substituted or unsubstituted benzene ring, substituted or unsubstituted heterocyclic aromatic ring, or substituted or unsubstituted naphthalene ring, R₃₃, R₃₄, R₃₅ and R₃₆ are independently H, amine, hydroxy, halide, acetyl, acetylamino, substituted or unsubstituted straight chain or branched alkyl, thioalkyl, aminoalkyl, oxyalkyl of 1 to 6 carbon atoms, substituted or unsubstituted alkenyl of 2 to 6 carbon atoms, substituted or unsubstituted aryl or heteroaryl of 6 to 14 carbon atoms, or R₃₃ and R₃₄ together form a substituted or unsubstiruted benzene ring, substituted or unsubstituted heterocyclic aromatic ring, or a substituted or unsubstituted naphthalene ring; R₃₅ and R₃₆ together form a substituted or unsubstiruted benzene ring, substituted or unsubstituted heterocyclic aromatic ring, or a substituted or unsubstituted naphthalene ring; Z is S or O, A₃, A₄, A₅, B₃, B₄, and B₅ are independently C, O, N or S, when A₃, A₄, A₅, B₃, B₄ or B₅ is C or N then Ra₃, Ra₄, Ra₅, Rb₃, Rb₄ or Rb₅ are independently H, S, O, amino, substituted or unsubstituted straight chain or branched alkyl, thioalkyl, aminoalkyl of 1 to 6 carbon atoms, substituted or unsubstituted aryl or heteroaryl, when A₃, A₄, A₅, B₃, B₄, or B₅ is O or S then Ra₃, Ra₄, Ra₅, Rb₃, Rb₄ or Rb₅ is an electron pair.

For DW3 (examples of substituents)
include, but are not limited to:
- halogen =: Cl, F, Br
- amino =: NH₂, CH₃,NH, N(CH₃)₂
- thioaklyl =: SCH₃, SC₇H₅
- oxyalkyl =: OCH₃, OC₂H₅
- amidoalkyl =: 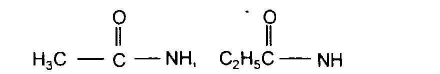
- aryl =: phenyl, benzyl
- thioaryl =: s-phenyl
- oxyaryl =: o-phenyl

For R₃₁ R₃₂ as above and also α, β napth
- aryl =: phenyl, 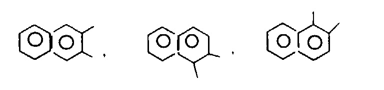
- heteroaryl =:

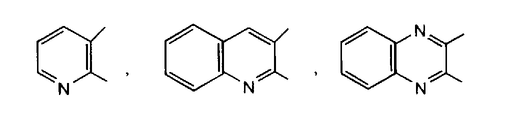

*Compounds designated DW4 have the structural formula:* 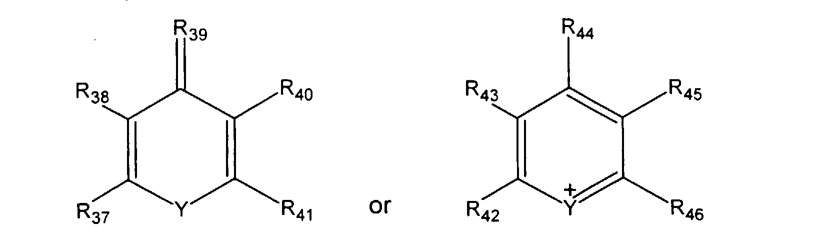 Y is Te or Se; R₃₉ is O, -C(CN)₂, R₃₇, R₃₈, R₄₀, R₄₁ are independently H, R₄₂, R₄₃, R₄₄, R₄₅, R₄₆, are independently H, R₄₄ is substitited or unsubstituted aryl, R₄₂ and R₄₃ together form a substituted or unsubstituted benzene ring, R₄₅ and R₄₆ together form a substituted or unsubstituted benzene ring.

For DW4
R₃₉ can also be NCCSO₂R;
wherein R can be:
R = methyl
R = phenyl

### Compositions

In one aspect, the present invention is directed to compositions comprising :
i) a haloperoxidase or haloperoxidase-labeled reactant, or a halide salt or pseudohalogen; or
ii) a haloperoxidase or haloperoxidase-labeled reactant and a halide salt or pseudohalogen; and
iii) a compound selected from DW1, or DW1 and DW1nuc, or DW2, or DW3, or DW4.
In another sense, the present invention relates to a composition prepared by combining :
i) a haloperoxidase or haloperoxidase-labeled reactant, or a halide salt or pseudohalogen; or
ii) a haloperoxidase or haloperoxidase-labeled reactant and a halide salt or pseudohalogen; and
iii) a compound selected from DW1, or DW1 and DW1nuc, or DW2, or DW3, or DW4.

### Dry Analytical Elements

In another aspect, the present invention relates to a dry analytical element comprising:
i) a spreading zone;
ii) one or more reagent zones; and
iii) a support;
wherein one or more of the zones comprises a compound selected from DW1, or DW1 and DW1nuc, or DW2, or DW3, or DW4.

### Mehods for Determining Analytes

In another aspect, the present invention relates to methods for determining, that is, detecting and/or quantifying analytes.

Accordingly, a method is provided for determining a peroxide comprising the steps of:
a) contacting a sample suspected of containing the peroxide with
   i) a compound from DW1, or DW1 and DW1nuc, or DW2, or DW3, or DW4,
   ii) a halide salt or pseudohalogen, and
   ii) a haloperoxidase, thereby producing a change in optical absorption; and
c) detecting the change in optical absorption as a measure of the amount of peroxide in the sample.

Dry analytical elements can be used for determining a peroxide. The dry analytical element comprises a spreading zone, one or more reagent zones and a support. The dry analytical element, optionally in any combination, further comprises a compound from DW1, or DW1 and DW1nuc, or DW2, or DW3, or DW4, a haloperoxidase, a halide salt or pseudohalogen; the method for determining peroxide comprising the steps of:
a) contacting the spreading zone of the dry analytical element with
   i) a sample suspected of containing the peroxide,
   ii) a halide salt or pseudohalogen if halide salt or pseudohalogen is not present in the element,
   iii) a haloperoxidase if haloperoxidase is not present in the element, thereby producing a change in optical absorption or reflection; and
b) detecting the change in optical absorption or reflection as a measure of the amount of peroxide in the sample.

In yet another aspect, the present invention relates to methods for determining a peroxide producing analyte comprising the steps of :
a) contacting a sample suspected of containing the peroxide producing analyte with,
   i) a reagent admixture capable of reacting with the analyte to produce peroxide,
   ii) a compound selected from DW1, or DW1 and DW1nuc, or DW2, or DW3, or DW4,
   iii) a haloperoxidase, and
   iv) a halide salt or pseudohalogen, thereby producing a change in optical absorption; and
b) detecting the change in optical absorption as a measure of the amount of analyte in the sample.

Peroxide producing analytes can also be determined using dry analytical elements.

Accordingly, a method is provided for determining a peroxide producing analyte using a dry analytical element. The dry analytical element comprises a spreading zone, one or more reagent zones and a support. The element, optionally in any combination, further comprises a haloperoxidase, a halide salt or pseudohalogen, and a reagent admixture capable of reacting with the analyte to produce peroxide; the method comprising the steps of:
a) contacting the spreading zone of the dry analytical element with
   i) a sample suspected of containing the peroxide producing analyte,
   ii) a halide salt or pseudohalogen if halide salt or pseudohalogen is not present in the element,
   iii) a haloperoxidase if haloperoxidase is not present in the element,
   iv) a reagent admixture capable of reacting with the analyte to produce peroxide, if said reagent admixture is not present in the element, thereby producing a change in optical absorption or reflection; and
b) detecting the change in optical absorption or reflection as a measure of the amount of peroxide producing analyte in the sample.

### Competitive and Sandwich Assays

The present invention is also directed to methods for performing competitive and sandwich assays using the signal-generating compounds of the present invention.

Accordingly, we provide a method for performing a competitive assay for an analyte comprising the steps of:
a) contacting a sample suspected of containing the analyte with
   i) an immobilized or immobilizable receptor specific to the analyte, thereby producing analyte that is bound and analyte that is not bound to the immobilized or immobilizable receptor,
   ii) haloperoxidase-labeled analyte, thereby producing haloperoxidase-labeled analyte that is bound and haloperoxidase-labeled analyte that is not bound to the immobilized or immobilizable receptor;
b) separating the haloperoxidase-labeled analyte that is bound from the haloperoxidase-labeled analyte that is not bound to the immobilized or immobilizable receptor;
c) contacting either the haloperoxidase-labeled analyte that is bound or the haloperoxidase-labeled analyte that is not bound to the immobilized or immobilizable receptor with,
   iii) a compound from DW1, or DW1 and DW1nuc, or DW2, or DW3, or DW4,
   iv) a halide salt or pseudohalogen, and
   v) a peroxide or peroxide generating agent, thereby producing a change in optical absorption; and
d) detecting the change in optical absorption as a measure of the amount of analyte in the sample.

A method for performing a competitive binding assay for an analyte using a dry analytical element also is provided, wherein the dry analytical element comprises a spreading zone, one or more reagent zones and a support. The dry analytical element further comprises an immobilized receptor specific to the analyte, and optionally in any combination, a compound from DW1, or DW1 and DW1nuc, or DW2, or DW3, or DW4, a halide salt or pseudohalogen, and haloperoxidase-labeled analyte, wherein the method comprises the steps of:
a) contacting the spreading zone of the dry analytical element with
   i) a sample suspected of containing the analyte, thereby producing analyte that is bound and analyte that is not bound to the immobilized receptor,
   ii) a haloperoxidase-labeled analyte if haloperoxidase-labeled analyte is not present in the element, thereby producing haloperoxidase-labeled analyte that is bound and haloperoxidase-labeled analyte that is not bound to the immobilized receptor;
b) separating the haloperoxidase-labeled analyte that is bound from the haloperoxidase-labeled analyte that is not bound to the immobilized receptor;
c) contacting the spreading zone of the dry analytical element with
   iii) a halide salt or pseudohalogen if halide salt or pseudohalogen is not present in the element, and
   iv) peroxide or peroxide-generating agent, thereby producing a change in optical absorption or reflection; and
d) detecting the change in optical absorption or reflection as a measure of the amount of analyte in the sample.

A method for performing a sandwich assay for an analyte is provided, the method comprising the steps of:
a) contacting a sample suspected of containing the analyte with
   i) an immobilized or immobilizable receptor specific to the analyte, thereby producing analyte that is bound and analyte that is not bound to the immobilized or immobilizable receptor,
   ii) a haloperoxidase-labeled receptor specific to the analyte, thereby producing haloperoxidase-labeled receptor that is bound and haloperoxidase-labeled receptor that is not bound to the immobilized or immobilizable receptor;
b) separating the haloperoxidase-labeled receptor that is bound from the haloperoxidase-labeled receptor that is not bound to the immobilized or immobilizable receptor;
d) contacting the haloperoxidase-labeled receptor that is bound to the immobilized or immobilizable receptor with
   iii) a compound selected from DW1, or DW1 and DW1nuc, or DW2, or DW3, or DW4,
   iv) a halide salt or pseudohalogen, and
   v) a peroxide or peroxide-generating agent, thereby producing a change in optical absorption; and
e) detecting the change in optical absorption as a measure of the amount of analyte in the sample.

An alternative method of performing a sandwich assay for an analyte is provided, comprising the steps of:
a) contacting a sample suspected of containing the analyte with
   i) an immobilized or immobilizable first receptor specific to the analyte, thereby producing analyte that is bound and analyte that is not bound to the immobilized or immobilizable first receptor,
   ii) a second receptor specific to the analyte, thereby producing second receptor that is bound and second receptor that is not bound to the immobilized or immobilizable first receptor;
   iii) haloperoxidase-labeled receptor specific to the second receptor, thereby producing haloperoxidase-labeled receptor that is bound and haloperoxidase-labeled receptor that is not bound to the immobilized or immobilizable first receptor;
b) separating the haloperoxidase-labeled receptor that is bound from the haloperoxidase-labeled receptor that is not bound to the immobilized or immobilizable first receptor;
c) contacting the haloperoxidase-labeled receptor that is bound to the immobilized or immobilizable first receptor with
   iv) a compound selected from DW1, or DW1 and DW1nuc, or DW2, or DW3, or DW4,
   v) a halide salt or pseudohalogen,
   vi) a peroxide or peroxide-generating agent, thereby producing a change in optical absorption; and
e) detecting the change in optical absorption as a measure of the amount of analyte in the sample.

Sandwich assays can also be conducted using dry analytical elements.

Accordingly, a method is provided for performing a sandwich assay for an analyte using a dry analytical element comprising a spreading zone, one or more reagent zones and a support. The dry analytical element further comprises immobilized receptor specific to the analyte, and optionally in any combination, a halide salt or pseudohalogen, a haloperoxidase-labeled receptor specfic to the analyte, a compound from DW1, or DW1 and DW1nuc, or DW2, or DW3, or DW4; the method comprising the steps of:
a) contacting the spreading zone of the dry analytical element with
   i) a sample suspected of containing the analyte, thereby producing analyte that is bound and analyte that is not bound to the immobilized receptor,
   ii) haloperoxidase-labeled receptor specific to the analyte, if the haloperoxidase-labeled receptor is not present in the dry analytical element, thereby producing haloperoxidase-labeled receptor that is bound and haloperoxidase-labeled receptor that is not bound to the immobilized receptor;
b) separating the haloperoxidase-labeled receptor that is bound from the haloperoxidase-labeled receptor that is not bound to the immobilized receptor;
c) contacting the spreading zone of the dry analytical element with
   iii) a compound from DW1, or DW1 and DW1nuc, or DW2, or DW3, or DW4 if the compound is not present in the dry analytical element,
   iv) a halide salt or pseudohalogen, if halide salt or pseudohalogen is not present in the dry analytical element,
   v) peroxide or peroxide-generating agent, thereby producing a change in optical absorption or reflection; and
d) detecting the change in optical absorption or reflection as a measure of the amount of analyte in the sample.

An alternative method of performing a sandwich assay for an analyte using a dry analytical element is also provided. The dry analytical element comprises a spreading zone, one or more reagent zones and a support. The dry analytical element further comprises immobilized first receptor specific to the analyte, and optionally in any combination, a halide salt or pseudohalogen, second receptor specfic to the analyte, haloperoxidase-labeled receptor specific to the second receptor, a compound from DW1, or DW1 and DW1nuc, or DW2, or DW3, or DW4; the method comprising the steps of:
a) contacting the spreading zone of the dry analytical element with
   i) a sample suspected of containing the analyte, thereby producing analyte that is bound and analyte that is not bound to the immobilized first receptor,
   ii) second receptor specific to the analyte if the second receptor is not present in the dry analytical element, thereby producing second receptor that is bound and second receptor that is not bound to the immobilized first receptor,
   iii) haloperoxidase-labeled receptor specific to the second receptor if the haloperoxidase-labeled receptor is not present in the dry analytical element, thereby producing haloperoxidase-labeled receptor that is bound and haloperoxidase-labeled receptor that is not bound to the immobilized first receptor;
b) separating the haloperoxidase-labeled receptor that is bound from the haloperoxidase-labeled receptor that is not bound to the immobilized first receptor;
c) contacting the spreading zone of the dry analytical element with
   iv) a compound selected from DW1, or DW1 and DW1nuc, or DW2, or DW3, or DW4 if the compound is not present in the dry analytical element,
   v) halide salt or pseudohalogen if halide salt or pseudohalogen is not present in the dry analytical element,
   vi) peroxide or peroxide-generating agent, thereby producing a change in optical absorption or reflection; and
d) detecting the change in optical absorption or reflection as a measure of the amount of analyte in the sample.

In any of the methods described above, incubation at a suitable temperature may be carried out to accelerate binding and/or chemical reactions. A useful temperature range includes about 20° C to about 100° C, and preferably about 30° C to about 40° C. The optical absorption or reflection change can be detected visually or with suitable instrumentation, such as a spectrophotometer or reflectometer.

The compositions described above can be dry, aqueous, organic, or a combination of aqueous and organic.

In the methods directed to competitive and sandwich assays any suitable procedure useful in a dry element or solution embodiment can be employed to separate bound and free haloperoxidase-labeled reactant, including washing, filtration or centrifugation, as are known in the art.

In preferred embodiments relating to compositions, dry analytical elements, and methods described hereinabove, the haloperoxidase is vanadium-bromoperxidase, the halide salt is a bromide salt, and the peroxide is hydrogen peroxide.

### Brief Description of the Figures

Figs. 1-6 show the absorption spectra that were obtained for vanadium bromoperoxidase catalysed bromination and nucleophilic coupling with specific compounds selected from DW1 and DW1nuc.

Fig. 7 shows the absorption spectra that were obtained for vanadium bromoperoxidase catalysed bromination of a specific compound selected from DW1 in the absence of a nucleophilic coupling compound.

Figs. 8-11 show the absorption spectra that were obtained for vanadium bromoperoxidase catalysed bromination of specific compounds selected from DW2.

Figs. 12-31 show the absorption spectra that were obtained for vanadium bromoperoxidase catalysed bromination of specific compounds selected from DW3.

Figs. 32 and 33 show the absorption spectra that were obtained for vanadium bromoperoxidase catalysed bromination of specific compounds selected from DW4.

FIG. 34 shows the absorption spectra that were obtained for vanadium bromoperoxidase catalysed bromination of phenol red.

Fig. 35 is a drawing showing the construction of a dry analytical element as in example 7.

Fig. 36 shows the measured tansmission intensity obtained at different levels of glucose using the dry element of example 7 comprising a compound from DW3.

### Detailed Description of the Invention

The present invention is used advantageously to determine the concentrations of analytes in various samples, such as human and animal biological fluids, foods, industrial or municipal effluents, and other fluids commonly tested in this manner. Biological fluids that can be tested include but are not limited to, whole blood, serum, plasma, urine, spinal fluid, lacrimal fluid, synovial fluid, lymphatic fluid, suspensions of tissues or plaque, gingival fluid, vaginal fluid, cranial fluid and other fluids readily apparent to one skilled in the art.

Analytes that can be determined include but are not limited to, mono, oligo and polysaccharides, trigylcerides, cholesterol esters, peptides, polypeptides, proteins (such as enzymes, antibodies, lipoproteins and glycoproteins), drugs, narcotics, steroids, toxins, nucleic acids and oligonucleotides, such as specific DNA or RNA sequences. More specifically, glucose, lactate, lipases, esterases, cholesterol, glycerol, uric acid, digoxin, phenytoin, phenobarbital, theophylline, C-reactive protein, human chorionic gonadotropin, thyroid stimulating hormone, thyroxine, triiodothyronine, creatine kinase-MB, carbamazepine, gentamicin, tobramycin or vancomycin, human immunodeficiency virus DNA or RNA, cytomegalovirus DNA or RNA, plant genomic DNA or RNA, human or other animal genomic DNA or RNA.

### Vanadium Bromperoxidase

The present invention is described in detail with vanadium bromoperoxidase, the preferred enzyme. However, this is only for illustrative purposes and is not to be construed as the only haloperoxidase that can be used in the practice of this invention.

Specific examples of proteinaceous vanadium bromoperoxidase sources that may be employed in the present invention can be found in Butler et al., Chem. Revs., 93, pp. 1937-1944, (1993) and include but are not limited to, those obtained from Ascophyllum nodosum, Ceramirum rubrum, Laminaria saccharina, Fucus distichus, Corallina pilulifera, Corallina officinalis, Macrocystis pyrifera. Procedures for isolation of vanadium bromoperoxidases may be found within the aforementioned Butler et al. reference. Of these proteinaceous vanadium bromoperoxidase sources, Ascophyllum nodosum is particularly preferred.

Analytical systems using vanadium bromoperoxidase and the signal-generating compounds of the present invention exhibit improved stability and enhanced detection without the need to add stabilizers, such as those described in U.S. Patent No. 5,372,931 to Friedman et al. or enhancers, as described in U.S. Patent No. 4,828,983 to McClune.

Vanadium bromoperoxidase (VBrPOD) is a more robust enzyme than HRP. VBrPOD tolerates both acidic and alkaline pH as well as neutral pH. VBrPOD functions in purely organic solvent systems (ethanol, methanol, carbon tetrachloride, dichloromethane) as well as in aqueous solvent systems (including water-miscible organic cosolvents) [Butler, A.; Carrano, C. J. *Coord. Chem. Rev.* 1991, 109, 61-105]. The turnover number of VBrPOD is quite high [Butler, A.; Carrano, C. J. *Coord. Chem. Rev.* 1991, 109, 61-105].

### Signal-Generating Compounds

In order to utilize a haloperoxidase, a compound is required that reacts with halogen or hypohalous acid to produce a signal change, and moreover, the compound must be highly selective for reaction with halogen or hypohalous acid relative to reaction with hydrogen peroxide.

A detectable signal can result from a change in a compound's electromagnetic radiation properties, for example, optical absorption, fluorescence, or refractive index. Preferably, the signal is due to an increase or decrease of its optical absorption in a region between approximately 300 and 800 nanometers and more preferably in a region between approximately 400 and 700 nanometers.

Unexpectedly, it has been found that the rate of oxidation of the signal-generating compounds of the present invention by hydrogen peroxide is slower than oxidation by halide /hypohalous acid by a factor having a range between approximately 10⁶ - 10⁸.

Direct addition of bromine or chlorine to chalcopyrilium dyes is known to produce a mixture of mono- and di-halogenated products because the second order rate constants for the formation of the mono- and di-halogenated products are similar (Detty, M.R. and Friedman, A.E., Organometallics, 1994, v13, pp 533-540). It was surprising, therefore, to find that the signal-generating compounds of the present invention in reaction with vanadium bromoperoxidase and halide ion form only one halogenated product or predominantly one halogenated product.

Specific examples of compounds in DW1 are: 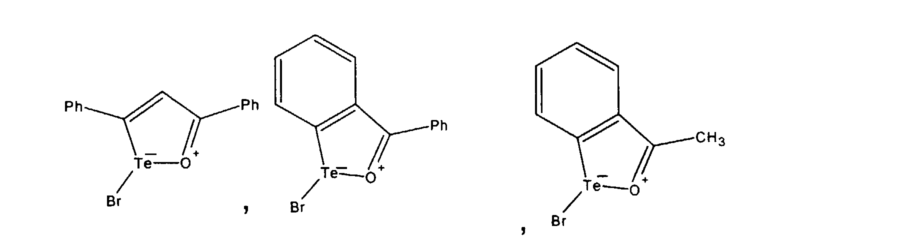 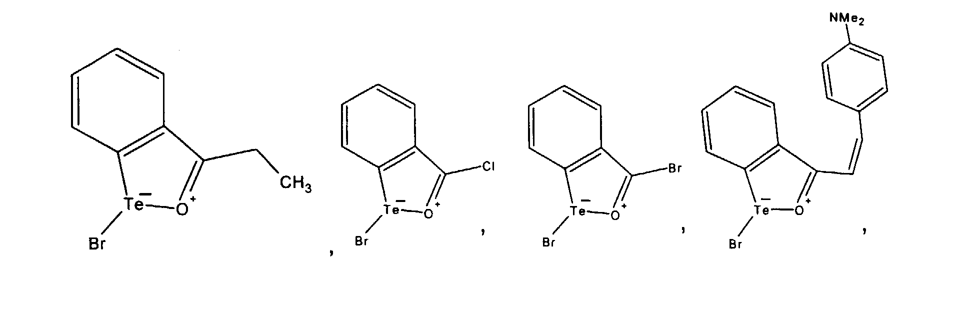 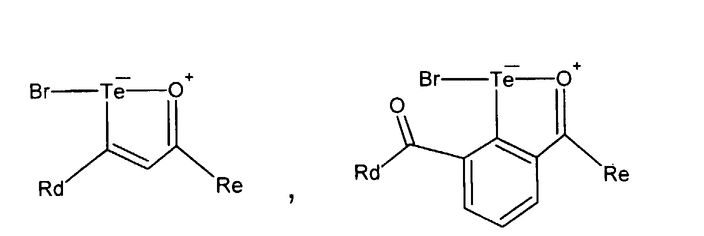 wherein Rd and Re are independently hydrogen, methyl, ethyl, propyl, chlorine, bromine, iodine, fluorine, or preferably

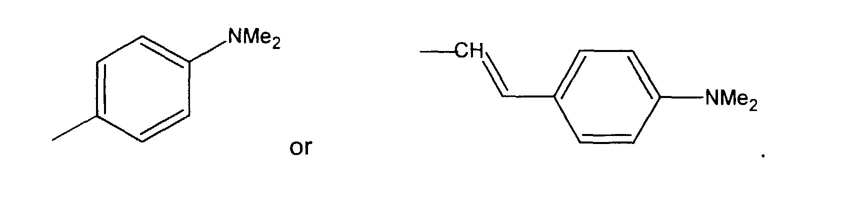

Specific examples of compounds in DW1nuc are:

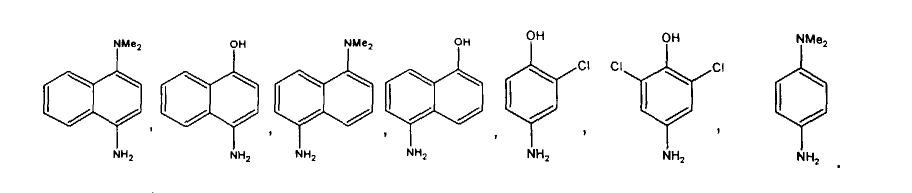

Specific examples of compounds in DW2 are: 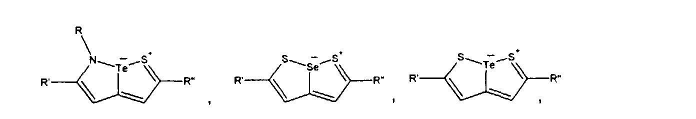 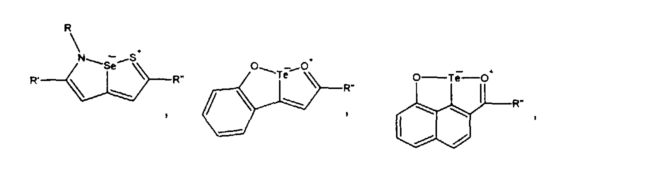 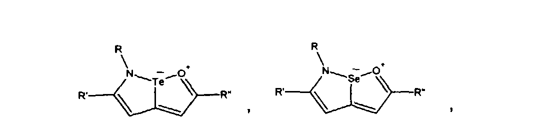 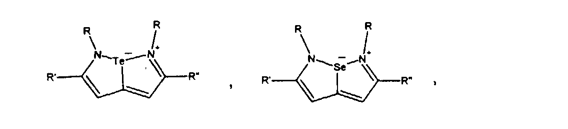 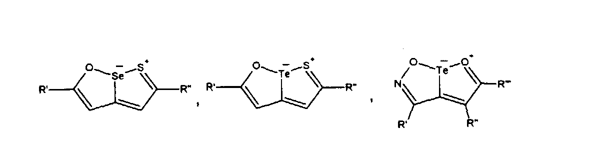 R is hydrogen, methyl or ethyl; R', R", and R"' are independently hydrogen, methyl, ethyl, vinylfuran, propyl, chlorine, bromine, iodine, fluorine, phenyl, halophenyl, nitrophenyl or preferably

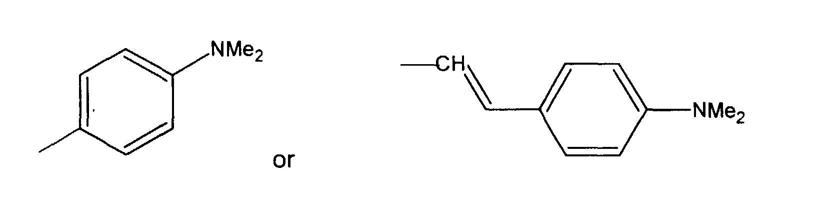

Examples of compounds in DW3 are: 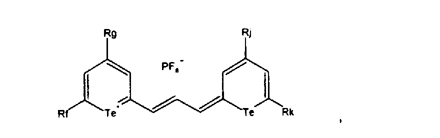 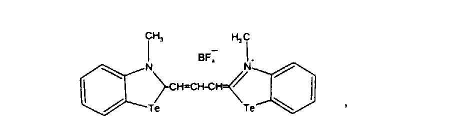 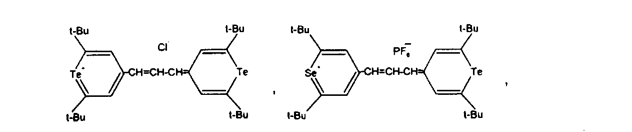 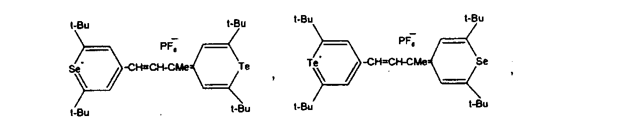 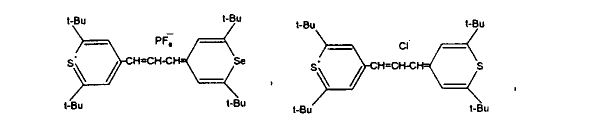 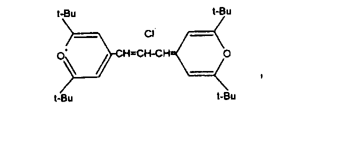 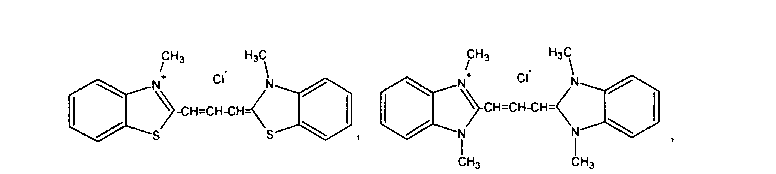 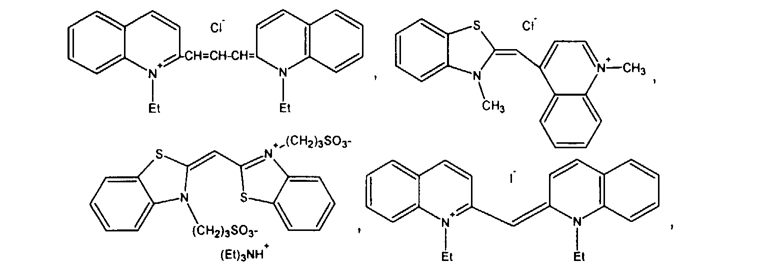 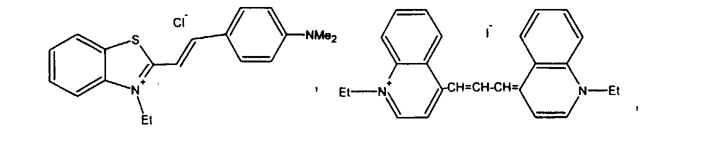 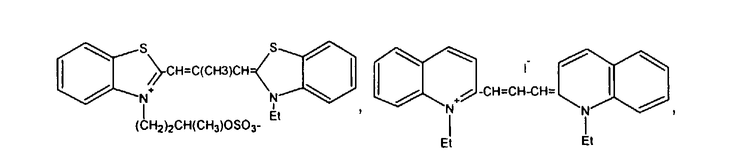 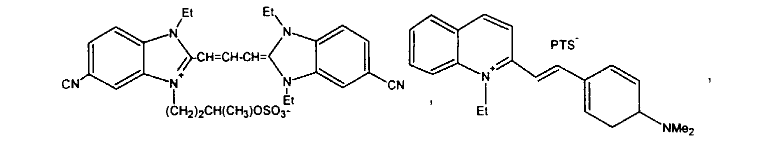 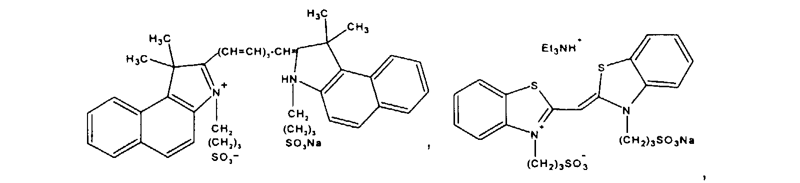 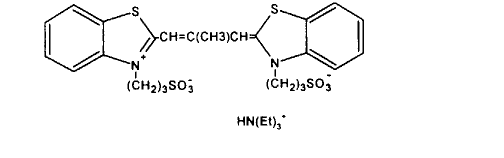 R_{f}, R_{g}, Rⱼ, Rₖ are independently hydrogen, substituted or unsubstituted straight chain or branched alkyl of 1 to 6 carbon atoms, oxyalkyl of 1 to 6 carbon atoms, substituted or unsubstituted alkenyl of 2 to 6 carbon atoms, substituted or unsubstituted aryl or heteroaryl of 6 to 14 carbon atoms.

Specific examples of compounds in DW4 are: 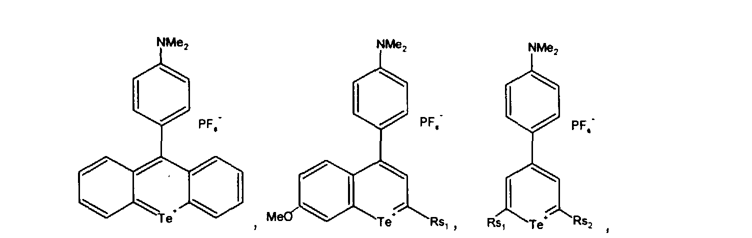 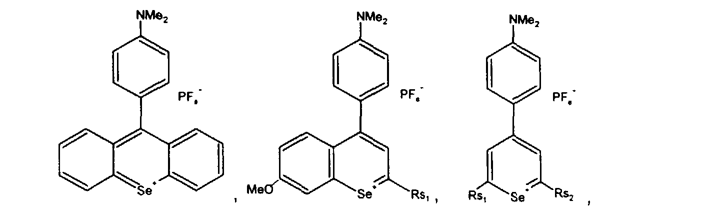 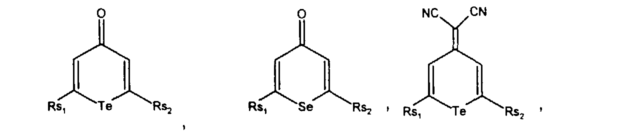 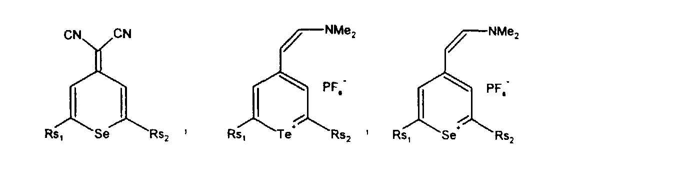 Rs₁ and Rs₂ are independently hydrogen, substituted or unsubstituted straight chain or branched alkyl of 1 to 6 carbons, such as methyl, halomethyl, aminomethyl, ethyl, haloethyl, aminoethyl, propyl, isopropyl, halopropyl, aminopropyl, butyl, isobutyl, tertiarybutyl, and the like, substituted or unsubstituted aryl or heteroaryl, such as phenyl, halophenyl, aminophenyl, naphthyl, halonaphthyl, aminonaphthyl, and the like.

It will be understood that a compound in any of DW1 through DW4 that carries a net positive or negative charge can be prepared and used with any counter-ion that will not interfere with its utility as a signal-generating agent. For example, if a compound carries a net positive charge suitable anionic counter-ions include but are not limited to, halide ion, CN⁻, PF₆⁻, BF₆⁻, ClO₄⁻, RCO₂⁻, or RSO₃⁻ wherein R is substituted or unsubstituted alkyl of 1 to 6 carbon atoms, or substituted or unsubstituted aryl or heteroaryl. If a compound carries a net negative charge, suitable cationic counter-ions include but are not limited to, Group IA metals such as Na⁺ and K⁺, Group IIA metal ions such as Mg⁺⁺ and Ca⁺⁺ or any other metal ion selected from the various Groups in the Periodic Table of the Elements. Cationic counter-ions may be non-metals such as, ammonium, aminoalkyl, aminoaryl, guanadinium or pyridinium, or other non-metal cations as are known in the art. Moreover, it is well known in the chemical arts how to prepare salts of compounds comprising any suitable counter-ion using, for example, ion exchange methods. Testing a particular signal-generating agent and counter-ion for suitable performance in an analytical application can be carried out readily and without undue experimentation, as would be well known to the skilled artisan. Briefly, the signal-generating compound and associated counter-ion are incorporated in the analytical system and the stability, sensitivity and any other related aspect of interest relative to desired performance are determined.

### Reaction of Compounds in DW1 with Nucleophiles in DW1nuc

Selenium(II) and tellurium(II) compounds are oxidized by halogen or hypohalous acid to form selenium (IV) and tellurium(lV) compounds at a much greater rate relative to their oxidation by H₂O₂. The tellurium(lV) or selenium(lV) intermediates function as Michael acceptors for unoxidized photographic developers such as para-phenylenediamines and para-aminophenols, such as those disclosed in James [James, T.H., *The Theory of the Photographic Process,* Fourth Edition, **1977**, 291-436].

The oxidative addition of either chlorine or bromine to compound **1** to produce compound **2** in Scheme **1** below occurs with an apparent second-order rate constant of ≥10⁸ M⁻¹ s⁻¹ at 295 K [Detty, M. R.; Luss, H.R.; McKelvey, J.M.; Geer, S. M. *J. Organic Chem.* 1986, 51, 1692]. In marked contrast, the oxidation of **1** with H₂O₂ is more than 100 million-fold slower, with a second-order rate constant of less than 1.0 M⁻¹ s⁻¹. Compound **2** is much more reactive toward nucleophiles than compound **1**, which via Michael addition followed by loss of a tellurium (IV), Te (IV), leaving group gives compound **3**. Such differences in rate are sufficient to permit development of registration systems for halogen and/or hypohalous acid in the presence of H₂O₂.

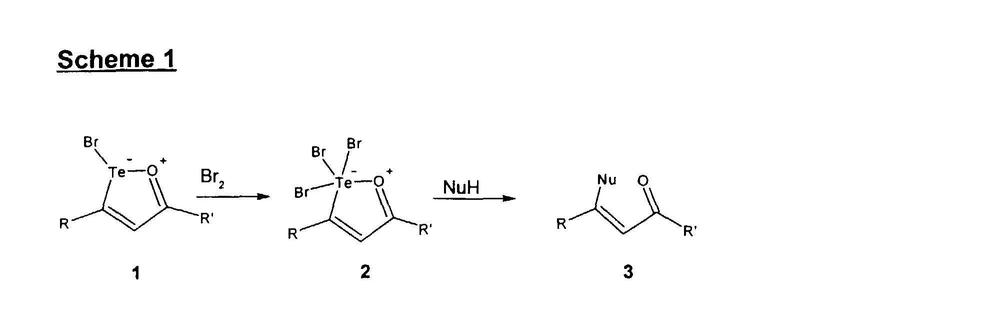

The chemical changes resulting in dye formation based on vanadium bromoperoxidase catalyzed bromination and coupling is illustrated more specfically with 4-amino-2,3-dichlorophenol as coupler (**Scheme 2**) and N,N-dimethyl phenylenediamine as coupler (**Scheme 3**).

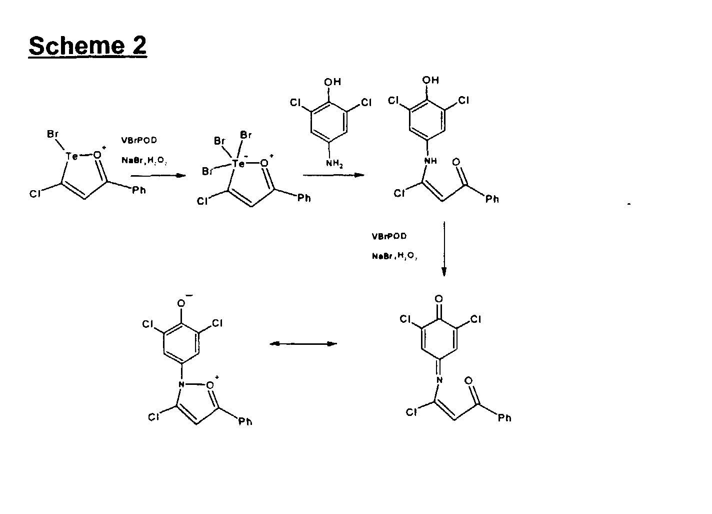

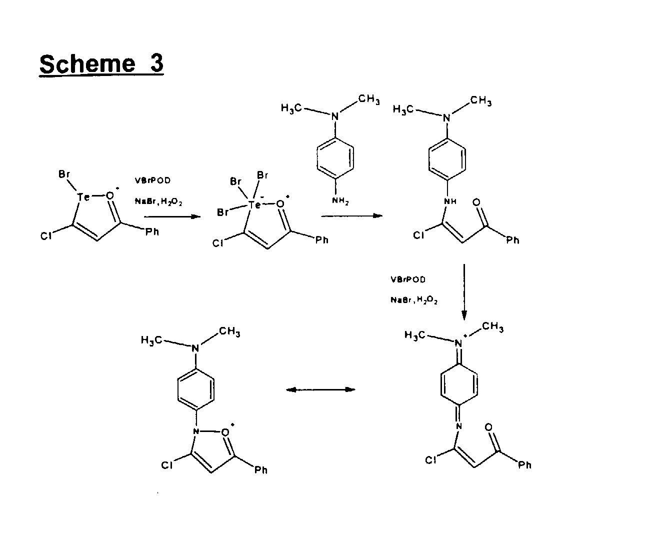

The structure of the compounds in DW1 can be modified for particular applications by appropriate selection of substituents. Aminoaryl or aminoheteroaryl substituents should lead to longer wavelength chromophores. Control of the size of the substituent groups as well as the electronic state of the ring will help tailor the rate of nucleophilic addition. The nature of the nucleophilic couplers will also determine the wavelength of absorption of the dyes that are formed, rates of oxidation, rates of nucleophilic addition, and dye stability. The chemistry described herein should be applicable to the generation of increased fluorescence or the generation of fluorescence chromophores as registration systems. The elimination of Te(lV) following Michael addition could enhance fluorescence through loss of a heavy atom (a fluorescence quencher).

Compounds in DW1 can serve directly as signal-generators without subsequent nucleophilic addition. The product that occurs upon oxidative halogenation of the Se or Te atom catalyzed by haloperoxidase (for example, as depicted in Scheme 1) can have a significantly different spectral absorption compared with the parent compound. This spectral difference can be used to detect and measure peroxide, haloperoxidase, halide anion, hypohalous acid, or other analytes. Although it may be desirable for some applications, it is not always necessary to provide for subsequent coupling with a nucleophile in order to obtain a detectable signal.

The dyes produced from the signal-generating compounds DW1, DW2, DW3 and DW4 do not readily fade under normal ambient lighting as do the prior art dyes such as those generated from di and triarylimidazoles upon HRP catalyzed oxidation. Moreover, the parent signal-generating compounds, DW1 through DW4, do not suffer from "color-up".

### Peroxide Producing Analytes

Various oxidoreductases, for example, galactose oxidase, glycollate oxidase, arylalcohol oxidase, bilirubin oxidase, cholesterol oxidase, glucose oxidase, glycerol oxidase, sorbose oxidase, and alcohol oxidase, directly catalyze oxidation of an enzyme specific substrate: galactose, glycollate, arylalcohol, etc. respectively, with concomitant reduction of oxygen to hydrogen peroxide. Other substances can be converted to an oxidase substrate using suitable coupling chemical reactions. For example, triglycerides and cholesterol esters can be hydrolyzed by enzyme hydrolases or bases (such as sodium hydroxide) to form glycerol and cholesterol, respectively. Glycerol and cholesterol are substrates, as indicated above, for their respective oxidases. Substances that can react to produce peroxide directly or indirectly (through suitable coupling chemistry, enzymatic, non-enzymatic or both) shall be known as peroxide producing analytes.

Peroxide producing analytes are capable of being detected and/or quantified using the present invention. The reagents necessary to produce peroxide from an analyte whether directly or indirectly and whether by enzyme-based or non-enzyme based chemical reactions or both shall be known as a reagent admixture capable of reacting with an analyte to produce peroxide.

### Dry Analytical Elements

The dry analytical elements discussed herein, comprise a porous spreading zone, usually a coated layer having suitable porosity for accommodating a liquid test sample. The element is assembled using techniques that are well known in the art. Preferably, the porous spreading zone is isotropically porous, which property is provided by interconnected spaces among the particles, fibers or other physical components of the zone. By isotropically porous is meant that fluids spread uniformly throughout the zone. Useful materials for such zones are water-insoluble and maintain their structural integrity during the assay.

Conventional materials and means for assembling a dry analytical element are described, for example, in U.S. Patent No. 3,992,158 to Przybylowicz et al., U.S. Patent No. 4,258,001 to Pierce et al., U.S. Patent No. 4,292,272 to Kitazima et al. and U.S. Patent No. 4,430,436 to Koyama et al. Preferred porous spreading zones are prepared from organopolymeric particles and a polymeric adhesive in a coherent, three-dimensional structure, as described in U.S. Patent No. 4,258,001 to Pierce et al.

The dry analytical elements, *inter alia,* can comprise any combination of, buffers, surfactants, organic salts, inorganic salts, synthetic polymers, natural polymers, proteins, haloperoxidase, haloperoxidase-labeled reactant (that is, haloperoxidase-labeled receptor, or haloperoxidase-labeled analyte), reagent admixture capable of reacting with an analyte to produce peroxide, and non-immobilized or immobilized receptor specific to an analyte.

There can be one or more additional zones in the element, which are in fluid contact with the porous spreading zone. The term "fluid contact" is used herein to denote that fluids can readily move from one zone to another. Such additional zones, preferably coated polymer layers, include subbing, reagent, and radiation blocking zones and are composed of one or more hydrophilic binder materials as are known in the art, such as gelatin, acrylamide polymers and vinylpyrrolidone polymers. Some zones may be water-insoluble while others may be water-soluble.

The zones of the element can be self-supporting, but preferably, these zones are disposed on a suitable dimensionally stable, chemically inert support. Preferably, the support is nonporous and transparent to electromagnetic radiation. A support of choice should be compatible with the intended mode of detection (for example, transmission, reflection, or fluorescence spectroscopy). Useful support materials include but are not limited to, paper, metal foils, polystyrenes, polyesters, polycarbonates and cellulose esters.

Additional details relating to dry multizone analytical elements, spreading zones, etc. may, for example, be found in U.S. Patent Nos. 3,867,258, 4,042,435, 4,050, 898, 4,066,403 and 4,153,668.

Optionally, the dry analytical element can comprise an absorbent material in fluid contact with the zones of the element. Absorbent materials provide overall added liquid capacity to the element, providing a reservoir for wash fluid. This is particularly advantageous for dry analytical elements directed to competitive or sandwich assays. Suitable absorbent materials include but are not limited to, glass microfibers, papers, sponges, fabrics, plastics and the like, so long as the material is capable of absorbing liquid.

If the dry analytical element does not comprise a combination of halide salt or pseudohalogen, haloperoxidase, reagent admixture capable of reacting with the analyte to produce peroxide, or signal-generating compound, any component of the combination that is not present in the element (or present in insufficient amount), and which is required for detecting the analyte, may be introduced during the assay by contacting the spreading zone of the element with the omitted component or components separately or in any other suitable combination prior to, with, or subsequent to the sample to be analyzed.

A preferred dry analytical element directed to a binding assay includes a receptor zone comprising an immobilized receptor specific to the analyte of interest. In a preferred competitive assay embodiment, haloperoxidase-labeled analyte is present in a label zone. Receptor zones and label zones prepared using a gravure coating technique are disclosed in U.S. Patent No. 5,714,340 to Sutton et al. and EP-A-0 740 157.

One method for preparing a receptor zone in a dry analytical element includes dispersing an immobilized receptor in a water soluble polymer(s) as described in the above-cited '340 patent to Sutton et al. Methods for immobilizing receptors are well known and examples may be found in EP-A-0 740 157 noted above and U.S. Patent No. 5,516,645 to Daniel et al. In brief, immobilized receptor specific to an analyte is generally fixed to particles that are dispersed throughout a zone of the element. Such particles can be composed of any suitable material including, but not limited to: glass, iron oxides, ceramics, synthetic or naturally occurring polymers, and have an average particle size of from about 0.01 to about 10 µm. Preferably, the particles are prepared from synthetic polymers and have suitable surface groups for adsorption or covalent attachment of the reactant molecules. A wide variety of such materials are known in the art such as those described in U.S. Patent No. 4,828,978 to Warren et al., U.S. Patent No. 4,997,772 to Sutton et al., U.S. Patent No. 5,147,777 to Sutton et al. or U.S. Patent No. 5,177,023 to Sutton et al., and references identified therein. Particularly useful polymeric particles are those prepared from ethylenically unsaturated polymerizable monomers having reactive carboxy, 2-substituted ethylsulfonyl or vinylsulfonyl groups, as described in the aforementioned Sutton et al. patents.

In analytical assays, haloperoxidase and signal-generating compounds may be added at levels suitable for the particular application. Optimal levels may be determined readily by introducing known amounts of haloperoxidase or haloperoxidase-labeled reactant, signal-generating compound, halide salt and any other appropriate reagents, into the dry analytical test element during preparation of the element. Alternatively, as noted above, omitted but required assay components may be introduced at the time of assay prior to, with, or subsequent to a test sample, until the desired signal intensity, reproducibility, analyte detection range, or other desired property is obtained.

The amount of haloperoxidase-labeled reactant can vary widely due to the amount of the other components used in the reaction, the suspected amount of analyte in the test sample, whether the assay is a competive or sandwich binding assay. Generally, the amount present in a dry analytical element is at least about 0.01 mg/m², with the preferred range being from about 0.1 to about 100 mg/m².

When present in a dry analytical element, haloperoxidase-labeled analyte can be located in any zone of the element. However, in competitive binding assay elements it is preferably not located in the same zone as the immobilized receptor, in order to minimize or prevent pre-binding of labeled-analyte to the immobilized receptor; haloperoxidase-labeled analyte is preferably present in a separate label zone. It is to be noted, however, that for some competitive binding assays pre-binding is not problematic and immobilized receptor and haloperoxidase labeled-analyte can be in the same zone without deleterious effect on assay performance. When required, these components can be separated by locating them in different zones of the element or they may be in the same zone but segregated by encapsulation of one or the other or both within a material that releases reactant at an appropriate time.

In sandwich binding assay elements, haloperoxidase labeled-receptor and immobilized receptor or unlabeled receptor and immobilized receptor may be in the same zone because pre-binding is not generally problematic.

Methods for linking analytes to enzymes, such as peroxidases, are well known and provided for example in U.S. Patent No. 5,298,403 to Danielson et al.

A preferred dry analytical sandwich assay element comprises immobilized receptor specific to the analyte of interest, vanadium bromoperoxidase-labeled receptor specific to the analyte, a bromide salt and a signal-generating compound of the present invention.

A preferred alternative sandwich assay element comprises immobilized receptor specific to the analyte of interest, second receptor specific to the analyte, vanadium bromoperoxidase-labeled receptor specific to the second receptor, a bromide salt and a signal-generating compound of the present invention.

If the element does not comprise a halide salt or pseudohalogen, second receptor (alternative sandwich assay) or haloperoxidase-labeled reactant (that is, labeled-analyte or labeled-receptor), each omitted component, as stated earlier, may be introduced into the element during the assay, separately or in any other suitable combination by contacting the spreading zone of the element prior to, with, or subsequent to the sample.

Separation of bound and free haloperoxidase-labeled reactant may be accomplished in any suitable manner. A preferred method involves contacting the spreading zone of the element with an aqueous composition (a wash solution) that contains peroxide or peroxide-generating agent and optionally, a buffer, a surfactant or any other necessary or useful reagent. Water without any added substances may also be used to effect separation.

A wash solution comprising peroxide or peroxide-generating agent, in addition to effecting separation of bound and free haloperoxidase-labeled reactant, initiates the chemical reactions required to produce detectable signal.

The wash solution (from about 5 to about 200 µL) may be applied in any suitable manner known in the art, but preferably, it is applied at a continuous metered rate up to about 10 µL/sec and most preferably at about 1 µL/sec. However, any rate and method of wash solution application may be used as long as the porous spreading zone readily absorbs the fluid during application. The method of application may comprise multiple fluid compositions applied sequentially.

### Halide Salt and Pseudohalogen

The halide salt may be a metal or non-metal salt. Non-metal salts include but are not limited to, ammonium, alkylammonium, substituted alkylammonium, arylammonium, substituted arylammonium, hydrazinium, oxonium, tropylium, sulfonium and the like. The halide salt may also be derived from a homopolymer or copolymer containing any combination of the above cationic species and halide anion as counter-ion. A preferred non-metal salt is ammonium bromide. Typically, the halide salt is a Group IA metal salt. Other metal salts in addition to Group IA metal salts are also contemplated herein. In a highly preferred embodiment the halide salt is sodium bromide.

In the practice of this invention, a "pseudohalogen" also may be employed. A pseudohalogen is any substance that provides anions having a high electron affinity comparable to the halogens. The pseudohalogen is typically coordinated to a Group IA metal. The pseudohalogen may be a metal or non-metal compound as described above for halides. Pseudohalogens include but are not limited to, salts of CN⁻, SCN⁻, OCN⁻, N₃⁻, and isonitriles. A preferred pseudohalogen is sodium thiocyanate.

### Peroxide and Peroxide-Generating Agent

As used herein, the term "peroxide" includes inorganic and organic peroxide compounds. A peroxide-generating agent is any reagent that is capable of producing a peroxide, such as perborates, peracetate and urea peroxide. Organic peroxides include, for example, peracids, peroxybenzoic acid, oxones, and hydroperoxides. Hydrogen peroxide is a preferred peroxide.

The following examples of signal-generating compounds and their reaction with vandium bromoperoxidase are provided to illustrate important aspects of this invention.

Except where noted, all reagents and equipment were obtained from commercial sources.

### Preparation of compounds in DW1

The syntheses of the tellurium-containing precursors has been reviewed in Detty, M. R. "Tellurium-Containing Heterocycles," *The Chemistry of Heterocyclic Compounds* series, E. C. Taylor, Ed.; Vol. 53, Wiley Interscience, New York: 1994.

### The nucleophiles DW1nuc are available commercially.

### Preparation of compounds in DW2

The syntheses of the tellurium-containing precursors has been reviewed in Detty, M. R. "Tellurium-Containing Heterocycles," *The Chemistry of Heterocyclic Compounds* series, E. C. Taylor, Ed.; Vol. 53, Wiley Interscience, New York: 1994.

### Preparation of compounds in DW3

The quaternary nitrogen compounds listed in columns aᵢ and bᵢ (i=1-10) of Table 1 were used to prepare the specific examples of DW3 compounds provided below.

As is known to the skilled synthetic chemist, quaternary compounds of the type used to synthesize compounds in DW3, can be prepared by combining the free base (available commercially, for example, from Aldrich Chemicals, Wisconsin) with either propane sultone, methyl iodide, ethyl iodide, or iodoethanol in acetonitrile, heating the mixture under reflux for 18 hours, and filtering the solid quaternary salt that is formed. This is the method that was used to prepare the quaternary compounds listed in Table 1. The solid quaternary salt obtained upon filtration was used directly to prepare the compounds from DW3, as described below. The starting compounds a₁₁ to a₁₃ used to prepare the ketomethylenes (preparations 11-13) were available commercially from Aldrich Chemicals. No structure was entered in column bᵢ in those cases where a symmetrical dye was prepared (i = 9-13).

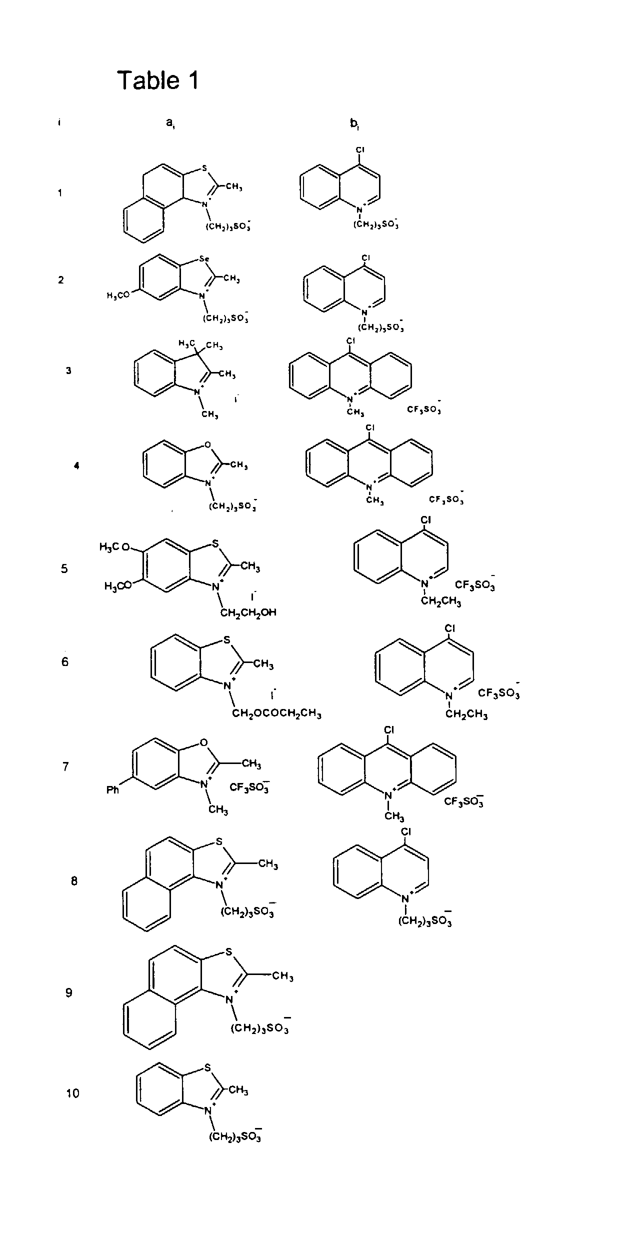

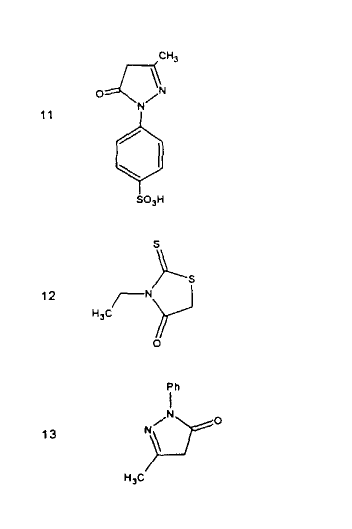

### 1. Preparation of:

Triethylammonium{3[4-((z)-1-[3-(1,1-dihydroxy-1-oxido-1-λ⁴-sulfanyl)propyl]naphtho[1,2-d][1,3]thiazol-1-ium-2-yl}methylidene)-1,4-dihydro-1-quinolinyl]propyl}(dihydroxy-λ⁴-sulfonate 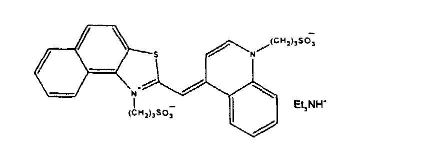 1.6 gm (0.005 moles) of a₁ and 1.4 gm (0.005 moles) of b₁ were combined with 100 mL ethanol and 4 mL of triethylamine (TEA). The mixture was heated to reflux for 2 hours, chilled, dithylether added and the precipitate was filtered, dried and recrystalized from ethanol. Product yield was 2.2 gm.

### 2. Preparation of:

Trihydroxy{3-[4-((E)-1-(5-methoxy-3-[3-(1,1,1-trihydroxy-λ⁴-sulfanyl)propyl]-1,3-benzoselenazol-3-ium-2-yl}methylidene)-1,4-dihydro-1-quinolinyl]propyl}-λ⁴-sulfane 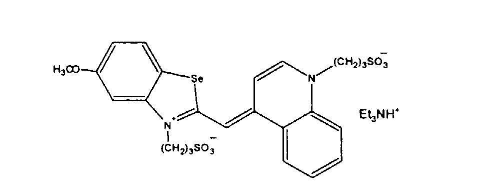 800 mg (2.3 millimoles) of a₂ and 580 mg (2 millimoles) of b₂ were added to 10 mL methanol and heated to reflux. One mL of TEA was added and the mixture was refluxed for 1 minute. One additional millilter of TEA was added and the mixture was refluxed again until all solids dissolved. The solution was heated for one additional minute then chilled on ice. Four mL of 5% NaOH was added - product began to precipitate. The mixture was chilled for 5 minutes, acetone added, the mixture filtered, and solid product recovered from the filtration was recrystalized by suspending and refluxing in acetonitrile with the addition of H₂O until all the solid dissolved.

### 3. Preparation of:

1,3,3-trimethyl-2-[(10-methyl-9,10-dihydro-9-acridinyliden)methyl]-3H-indolium iodide 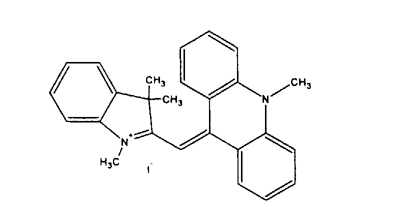 Equimolar amounts of a₃ and b₃ were combined in acetonitrile, heated to reflux. TEA was added, and the mixture was heated for 2 minutes, then chilled on ice. Acetone was added and the mixture was refrigerated overnight. The solid product was filtered and dried.

### 4. Preparation of:

Dihydroxy(3-2-[(10-methyl-9,10-dihydro-9-acridinyliden)methyl]-1,3-benzoxazol-3-ium-3-ylpropyl)-I4-sulfanolate 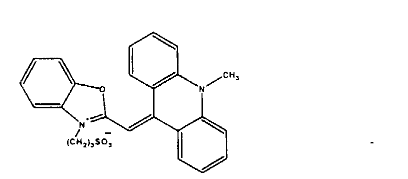 Equimolar amounts of a₄ and b₄ were combined in acetonitrile and heated to reflux. TEA was added and product formed immediately. The suspension was refluxed for 1 minute, chilled on ice, followed by addition of diethylether. The resulting gum was washed twice with diethylether. Acetone was added and the mixture was heated to reflux. The crystalline product was filtered and then recrystalized from methanol.

### 5. Preparation of:

2-5,6-dimethoxy-2-[(1-phenyl-1,4-dihydro-4-quinolinyliden)methyl]-1,3-benzothiazol-3-ium-3-yl-1-ethanol iodide 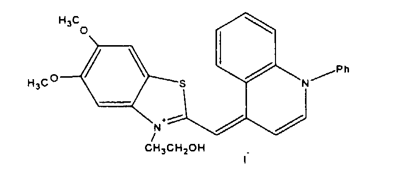 Equimolar amounts of a₅ and b₅ were combined in acetonitrile. TEA was added and product formed immediately. The suspension was heated to reflux for 30 seconds, chilled on ice and the solid product was filtered. The product was recrystalized by suspending it in methanol, heating to reflux, adding water and chilling on ice. The solid was filtered, washed with acetone and dried at 100°C for six hours.

### 6. Preparation of:

Ethyl 2-2-[(1-ethyl-1,4-dihydro-4-quinolinyliden)methyl]-1,3-benzothiazol-3-ium-3-ylacetate tetrafluoroborate 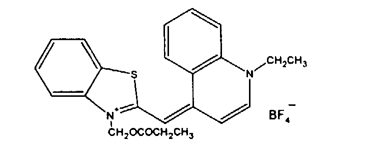 Equimolar amounts of a₆ and b₆ were combined in methanol and TEA was added. The mixture was heated to reflux for 30 seconds, and then cooled. An aqueous solution of sodium tetrfluoroborate was added and product precipitated. The solid was filtered, washed with methanol and recrystalized from a solution of ethanol and acetonitrile.

### 7. Preparation of:

3-methyl-2-[(10-methyl-9,10-dihydro-9-acridinyliden)methyl]-5-phenyl-1,3-benzoxazol-3-ium tetrafluoroborate 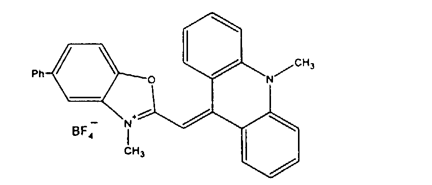 Equimolar amounts of a₇ and b₈ were combined in ethanol and TEA was added. The mixture was heated to reflux for 30 seconds, and then cooled. An ethanolic solution of tetrabutyl ammonium tetrfluoroborate was added and the product dye precipitated. The solid dye was filtered and recrystalized by suspending the solid in refluxing ethanol and additn acetonitrile until it dissolved. The mixture was chilled on ice, filtered and dried at at 100°C.

### 8. Preparation of:

Triethylammonium 3-[4-((Z)-1-1-[3-(1,1-dihydroxy-1-oxido-I4-sulfanyl)propyl]naphtho[1,2-d][1,3]thiazol-1-ium-2-ylmethylidene)-1,4-dihydro-1-quinolinyl]propyl(dihydroxy)-I4-sulfanolate 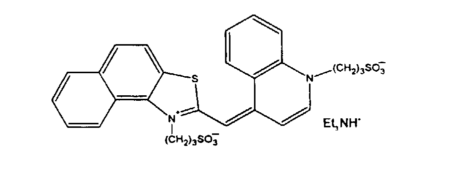 1.6 gm of a₈ and 1.5 gm b₈ were combined in 50 mL of absolute ethanol. Two mL TEA was added. The mixture was heated to reflux for 2 hours, and then chilled on ice. The solid dye was filtered and recrystalized from about 40 mL of ethanol.

### 9. Preparation of:

Triethylammonium 3-[2-((Z)-1-1-[3-(1,1-dihydroxy-1-oxido-I4-sulfanyl)propyl]naphtho[1,2-d][1,3]thiazol-1-ium-2-ylmethylidene)-1,2-dihydronaphtho[1,2-d][1,3]thiazol-1-yl]propyl(dihydroxy)-I4-sulfanolate 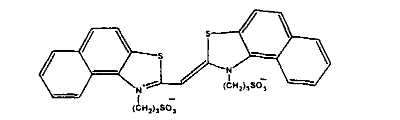 a₉ was suspended in dimethylformamide (DMF), TEA was added, followed by acetic anhydride (AC₂O) and then isopentyl nitrite (IPN). The mixture was heated to reflux. It turned yellow-green, gas evolved and dye began to precipitate. The suspension was heated to reflux for 5 minutes. Additional TEA, AC₂O and IPN were added. The mixture was held at reflux for 2 minutes, cooled to room temperature. The solid dye was filtered, recrystalized from methanol by filtering the hot mixture. The solid dye was dried at 100°C.

### 10. Preparation of:

Triethylammonium 3-[2-(3-[2-(1,1-dihydroxy-1-oxido-I4-sulfanyl)ethyl]-2,3-dihydro-1,3-benzothiazol-2-ylidenmethyl)-1,3-benzothiazol-3-ium-3-yl]propyl(dihydroxy)-I4-sulfanolate 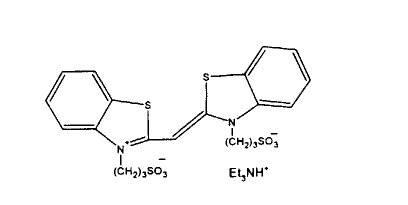 a₁₀ was suspended in acetonitrile and dimethyl was added, followed by acetic anhydride (AC₂O) and then isopentyl nitrite (IPN). The mixture was heated to reflux. It turned yellow-green, gas evolved and dye began to precipitate. The suspension was heated to reflux for 5 minutes. Additional TEA, AC₂O and IPN were added. The mixture was held at reflux for 2 minutes, cooled to room temperature. The solid dye was filtered and recrystalized from methanol; the soild was filtered from hot methanol. The dye was dried at 100°C.

### 11. Preparation of:

Di(triethylammonium)4-(4-(Z)-1-[5-hydroxy-3-methyl-1-(4-sulfonatophenyl)-1H-4-pyrazolyl]methylidene-3-methyl-5-oxo-4,5-dihydro-1H-1-pyrazolyl)-1-benzenesulfonate 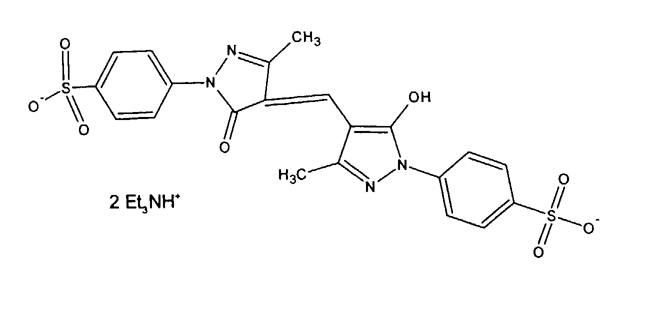 a₁₁ was suspended in acetonitrile. N,N-diisopropylethylamine and diethoxymethyl acetate were added. The suspension was heated to reflux until a solution formed. The solution was refluxed for 5 more minutes, cooled and the precipitated dye was filtered washed with acetonitrile. The dye was recrystalized from acetonitrile; solid dye was filtrered from the hot acetonitrile.

### 12. Preparation of:

Triethylammonium 3-ethyl-5-[(3-ethyl-4-oxo-2-thioxo-1,3-thiazolan-5-yliden)methyl]-2-thioxo-2,3-dihydro-1,3-thiazol-4-olate 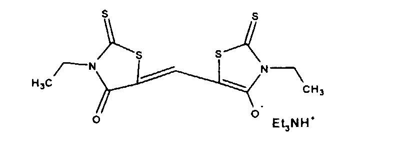 a₁₂ was heated in a mixture of acetonitrile, diethoxymethyl acetate and TEA. The mixture was refluxed for one-half hour, cooled, poured into diethylether. Dye crystalized from solution. The dye was recrystalized from a mixture of acetonitrile and ethanol.

### 13. Preparation of:

4-[(Z)-1-(5-hydroxy-3-methyl-1-phenyl-1H-4-pyrazolyl)methylidene]-3-methyl-1-phenyl-4,5-dihydro-1H-5-pyrazolone 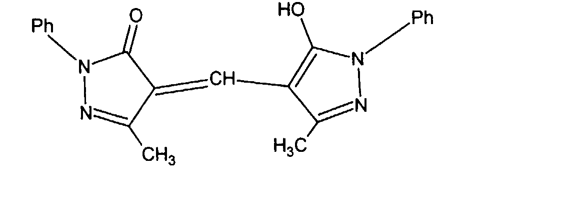 a₁₃ was heated in a mixture of acetonitrile, diethoxymethyl acetate and TEA. Solid dye precipitated and was recrystalized from acetonitrile.

### Preparation of compounds from DW4

The syntheses of the tellurium-containing precursors has been reviewed in Detty, M. R. "Tellurium-Containing Heterocycles," *The Chemistry of Heterocyclic Compounds* series, E. C. Taylor, Ed.; Vol. 53, Wiley Interscience, New York: 1994.

### Solution-Based Examples

### EXAMPLE 1

### Spectral absorption changes accompanying vanadium bromoperoxidase catalyzed bromination of specific compounds from DW1 and coupling with specific nucleophiles from DW1nuc.

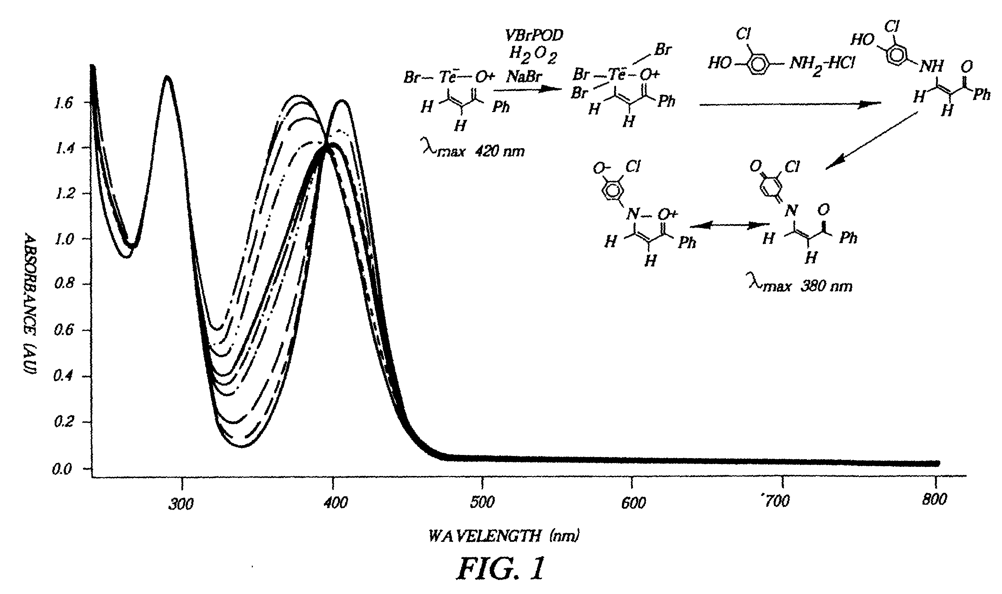

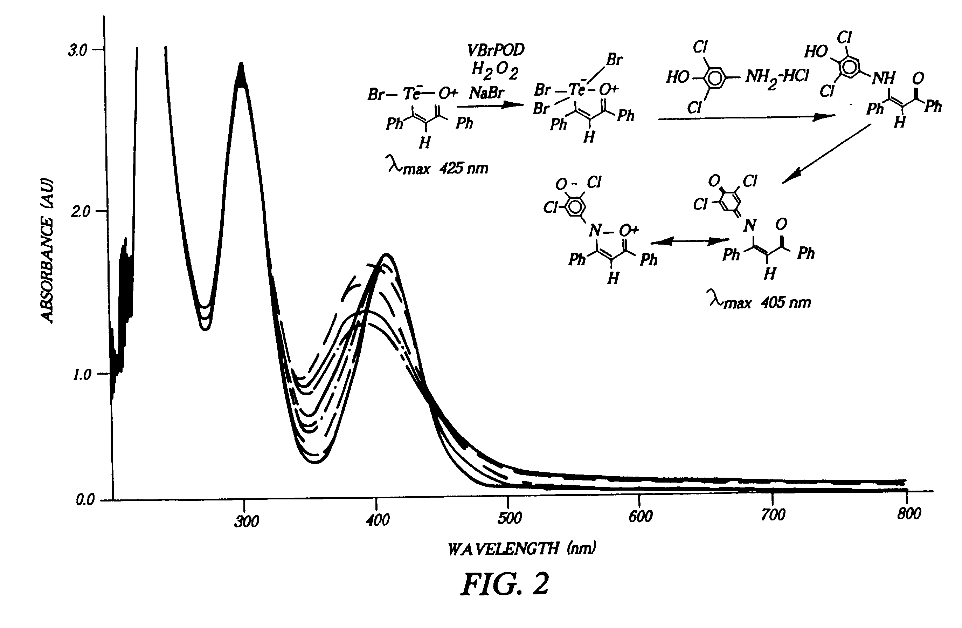

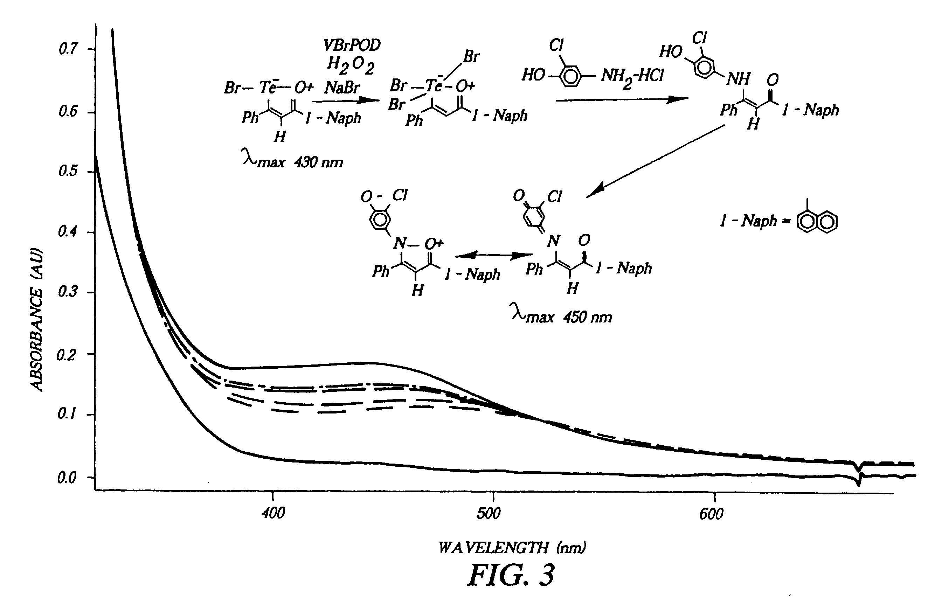

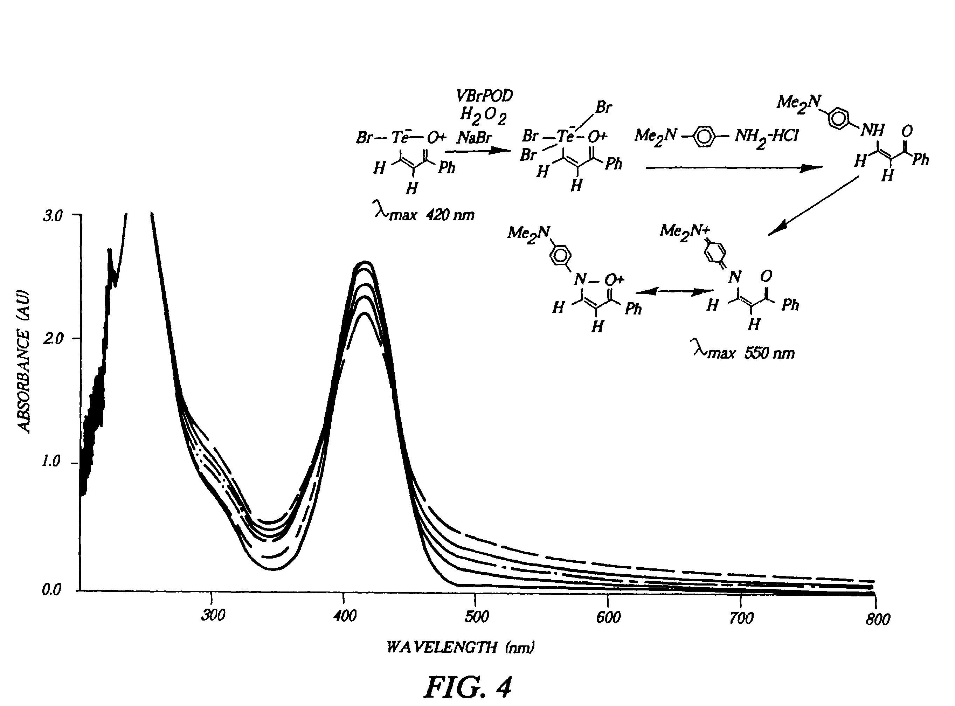

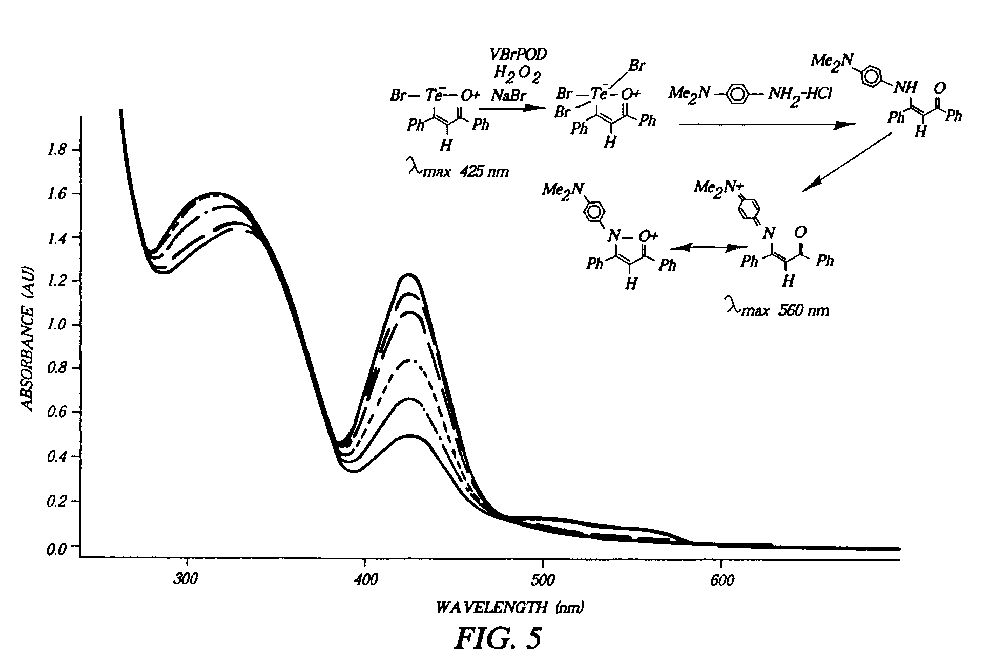

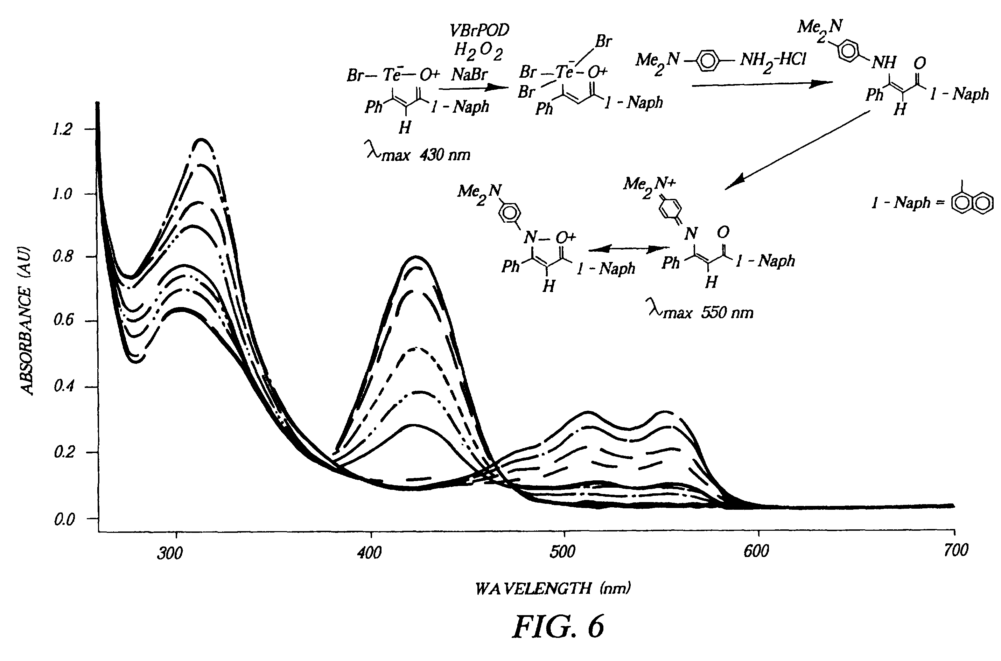

In Figures 1-6, 5X10⁻¹² - 5X10⁻¹¹ M vanadium bromoperoxidase was added to a solution of a tellurium and /or selenium containing compound (1 to
3X10⁻⁵ M) in 0.05 M phosphate buffer (pH 7) containing one of the nucleophiles, p-aminophenol, p-N,N-dimethylphenylenediamine, 2-chloro-4-aminophenol, or 2,6-dichloro-4-aminophenol as their hydrochloride salts (1 to 3X10⁻⁵ M), 0.025-0.10 M NaBr and 5 x 10⁻⁵ - 2.5 x 10⁻⁴ M H₂O₂ at ambient temperature in a 1-cm pathlength quartz cuvette. Spectra were recorded on a Hewlett-Packard diode array spectrophotometer at various times after addition of enzyme. Oxidation with bromine by the enzyme resulted in a decrease of absorption at the λₘₐₓ of the starting material and an increase of absorption at a different wavelength. The rate of change of absorption was a function of enzyme, peroxide, and bromide concentrations.

### EXAMPLE 2

### Spectral absorption changes accompanying oxidative bromination of a Te compound from DW1 catalyzed by vanadium bromoperoxidase.

As noted earlier, oxidative halogenation of a Se or Te compound from DW1 can be used in the absence of nucleophile to detect and/or quantitate an analyte if the halogenated product has spectral absorption characteristics different from the parent compound.

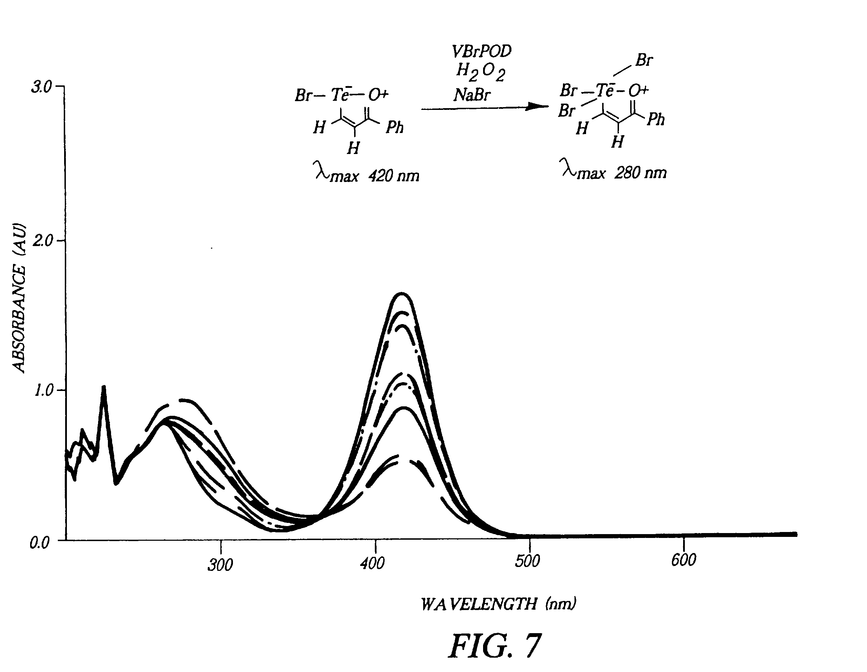

The Te compound of Figure 7 was oxididatively brominated in the presence of VBrPOD, H₂O₂ and NaBr. A 10⁻⁹ M stock solution of vanadium bromoperoxidase was added to a solution of the tellurium heterocycle (5 x 10⁻⁶ - 3 x 10⁻⁵ M) in 0.05 M phosphate buffer (pH 6.9) containing 0.045 M NaBr and 5 x 10⁻⁵ - 2.5 x 10⁻⁴ M H₂O₂ at ambient temperature in a 1-cm pathlength quartz cuvette. Spectra were recorded on a Hewlett-Packard diode array spectrophotometer at various times after addition of enzyme. Oxidation with bromine by the enzyme resulted in a decrease of absorption at the λmax of the starting material and an increase of absorption at a different wavelength. The rate of change of absorption was a function of enzyme, peroxide, and bromide concentrations.

### EXAMPLE 3

### Spectral absorption changes accompanying vanadium bromperoxidase catalyzed oxidation of tellurium and selenium heterocyclic compounds from DW2.

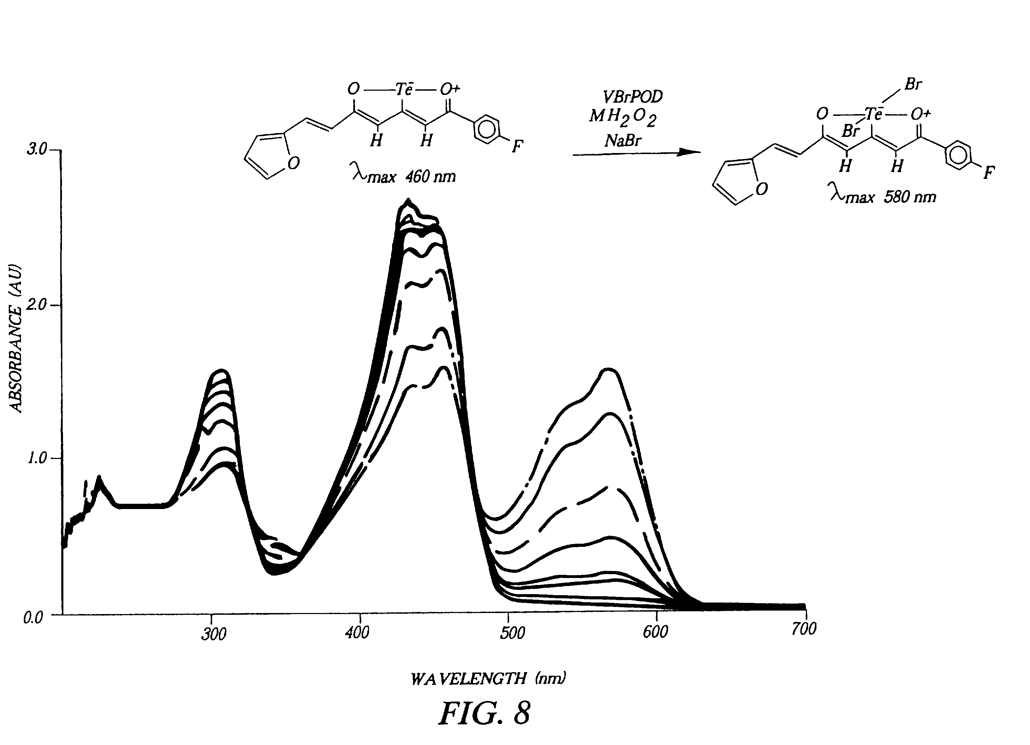

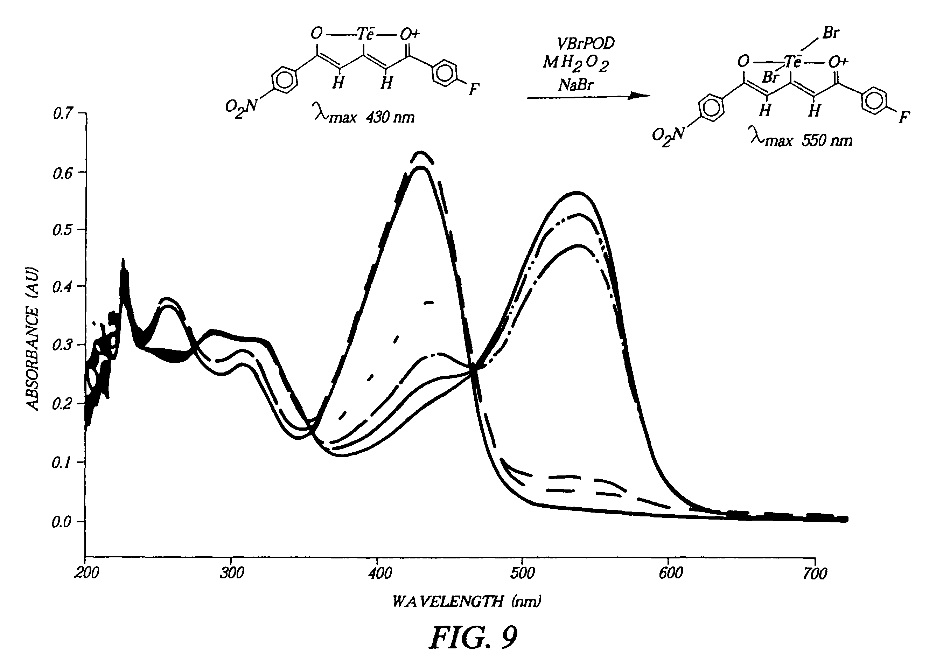

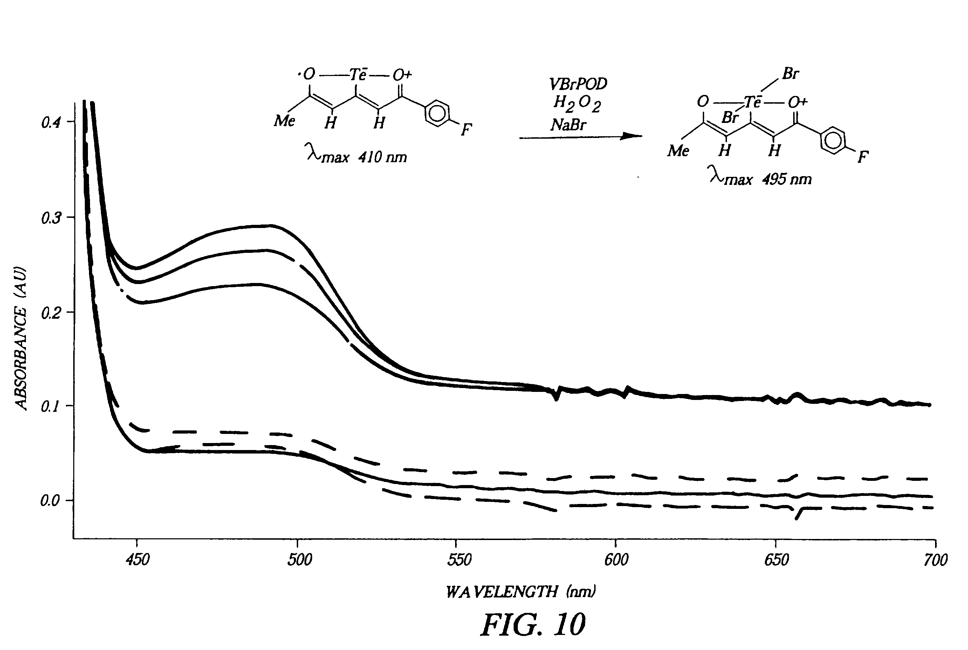

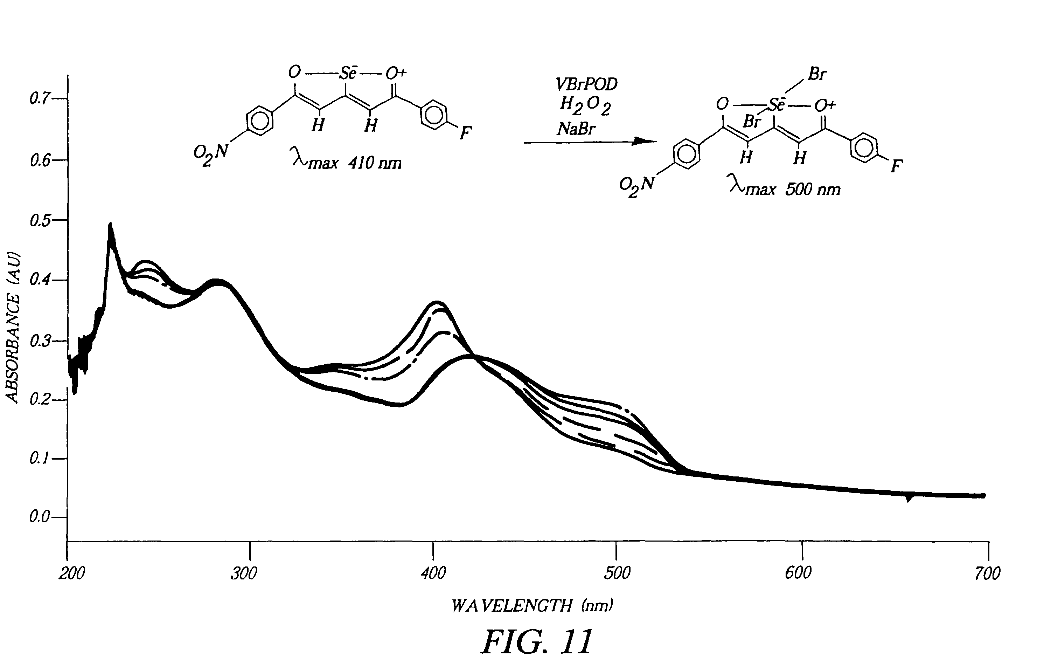

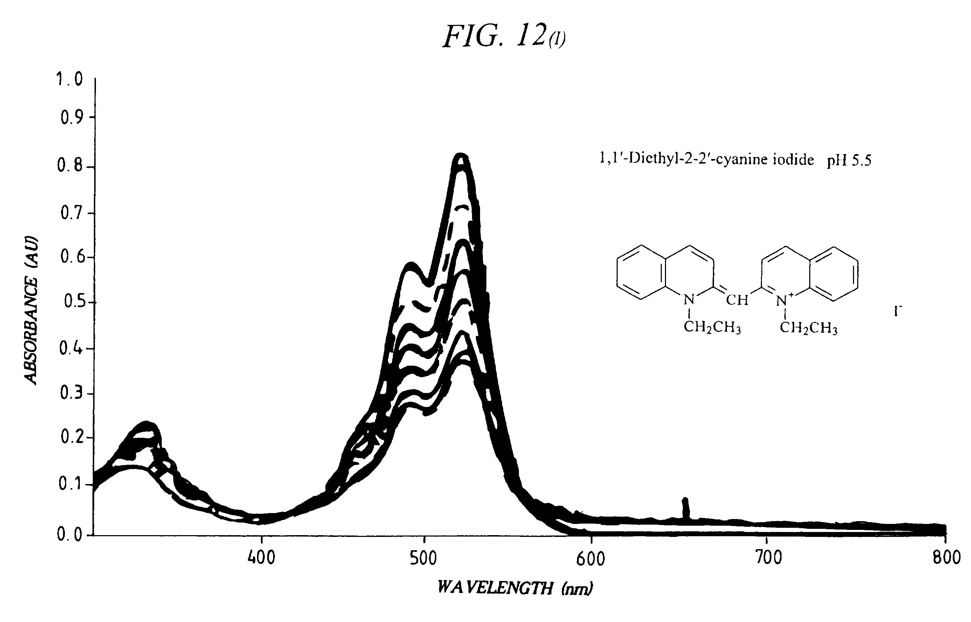

In each of the experiments shown in Figures 8-11, 10⁻⁹ M stock solution of vanadium bromoperoxidase was added to a solution of tellurium and/or selenium-containing heterocycle (5 x 10⁻⁶ - 3 x 10⁻⁵ M) in 0.05 M phosphate buffer (pH 6.9) containing 0.045 M NaBr and 5 x 10⁻⁵ - 2.5 x 10⁻⁴ M H₂O₂ at ambient temperature in a 1-cm pathlength quartz cuvette. Spectra were recorded on a Hewlett-Packard diode array spectrophotometer at various times after addition of enzyme. Oxidation with bromine by the enzyme resulted in a decrease of absorption at the λₘₐₓ of the starting material and an increase of absorption at a different wavelength. The rate of change of absorption was a function of enzyme, peroxide, and bromide concentrations.

### EXAMPLE 4

### Spectral absorption changes accompanying vanadium brompoeroxidase catalyzed bromination of compounds from DW3.

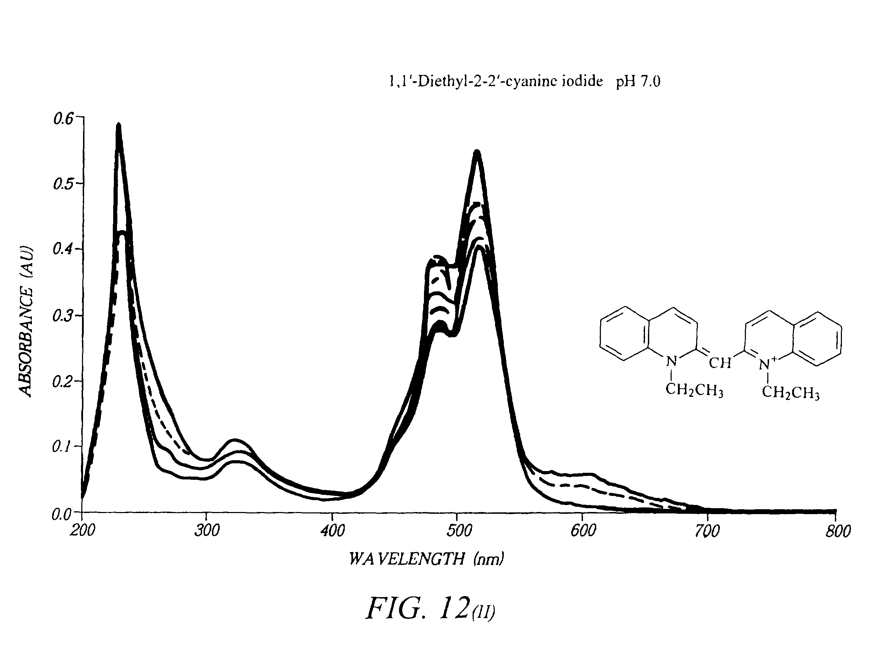

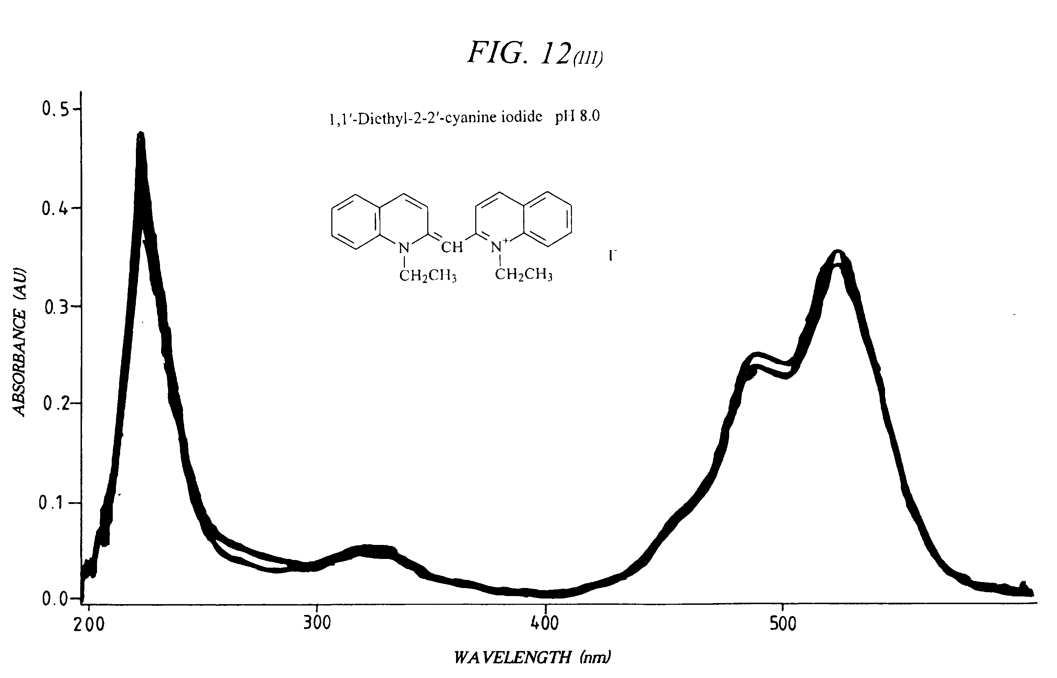

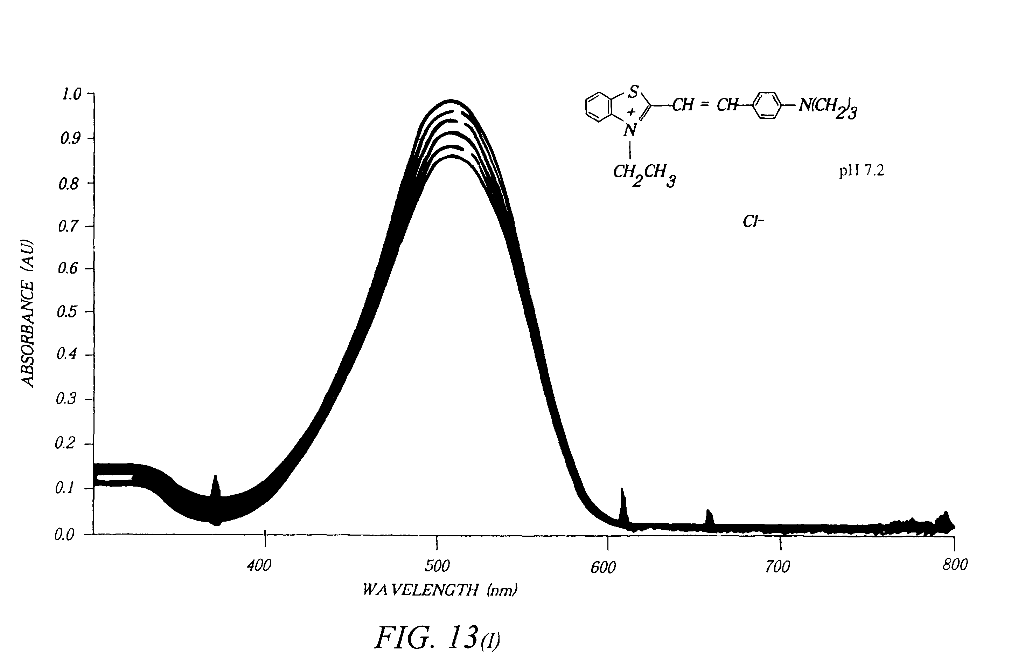

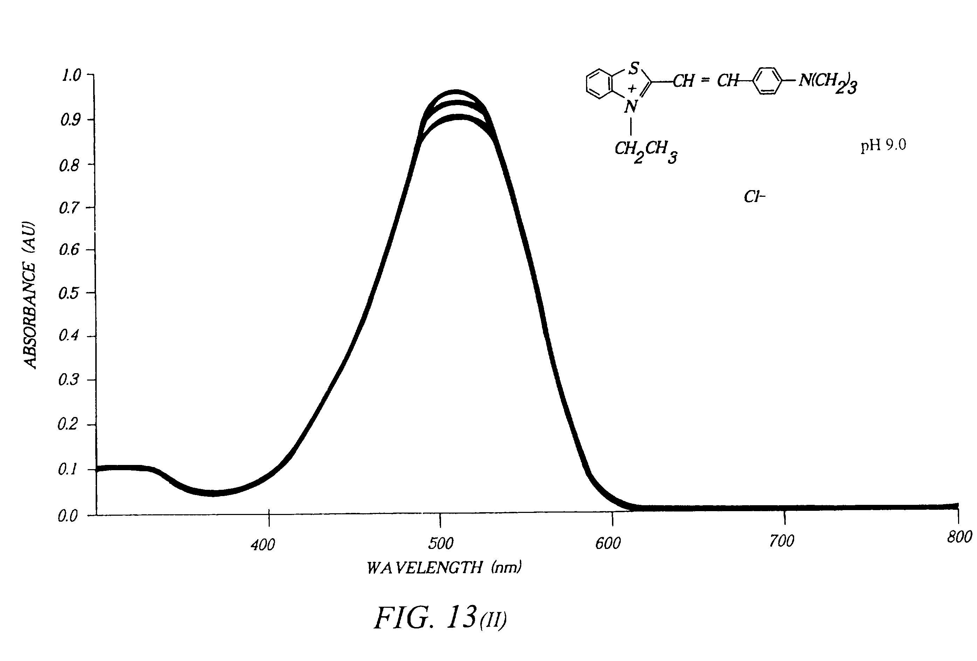

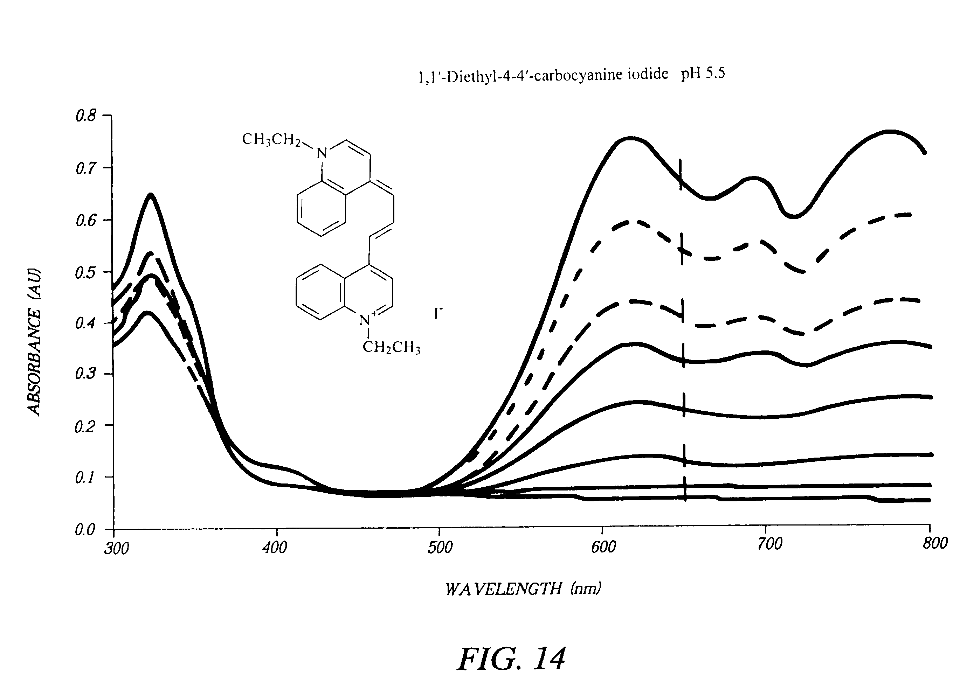

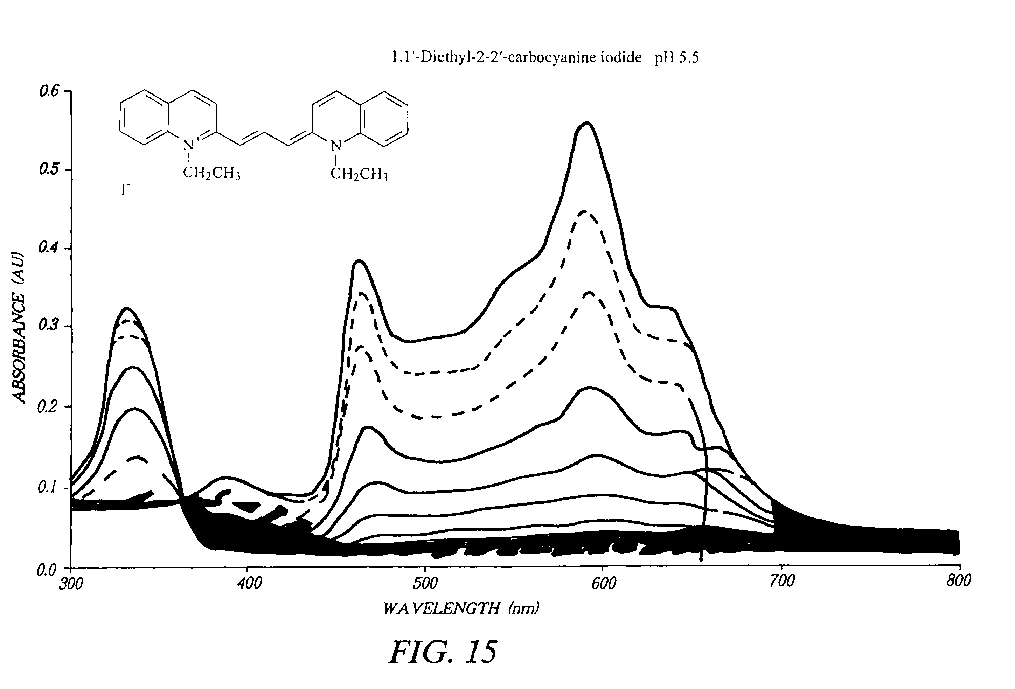

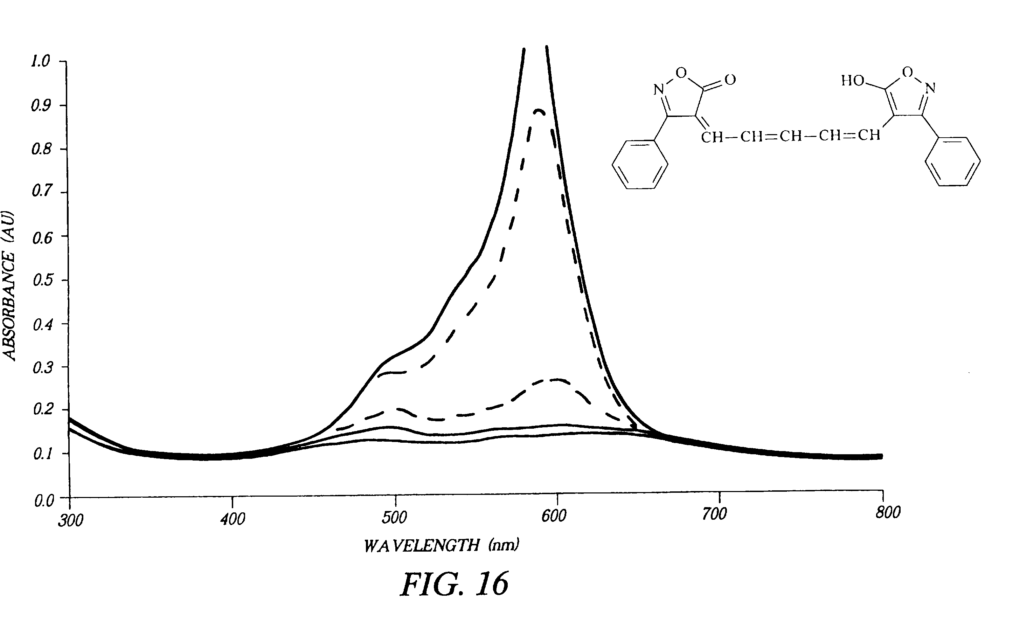

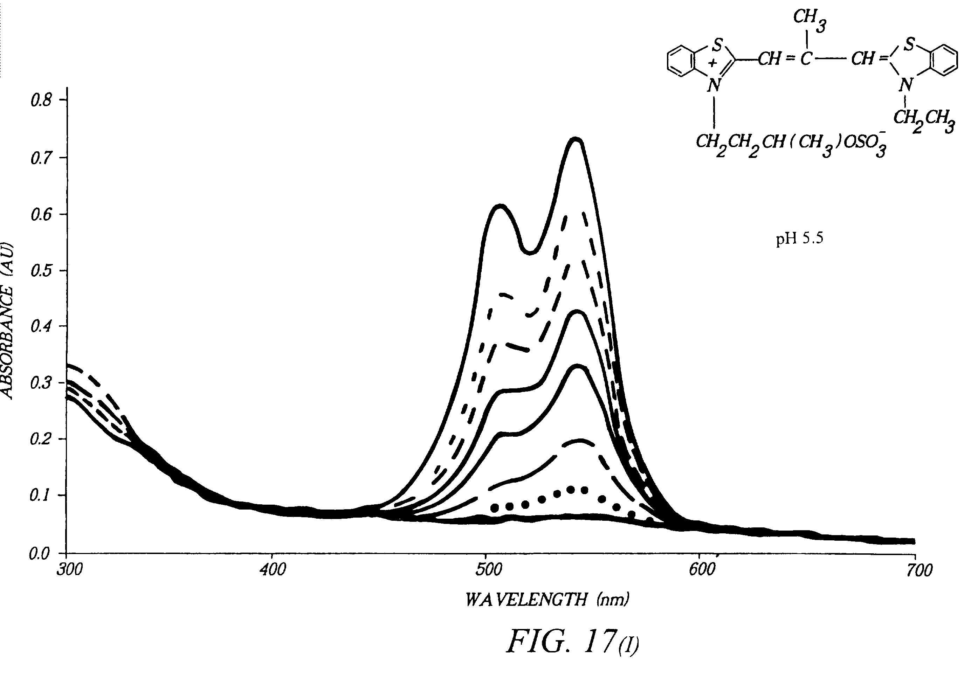

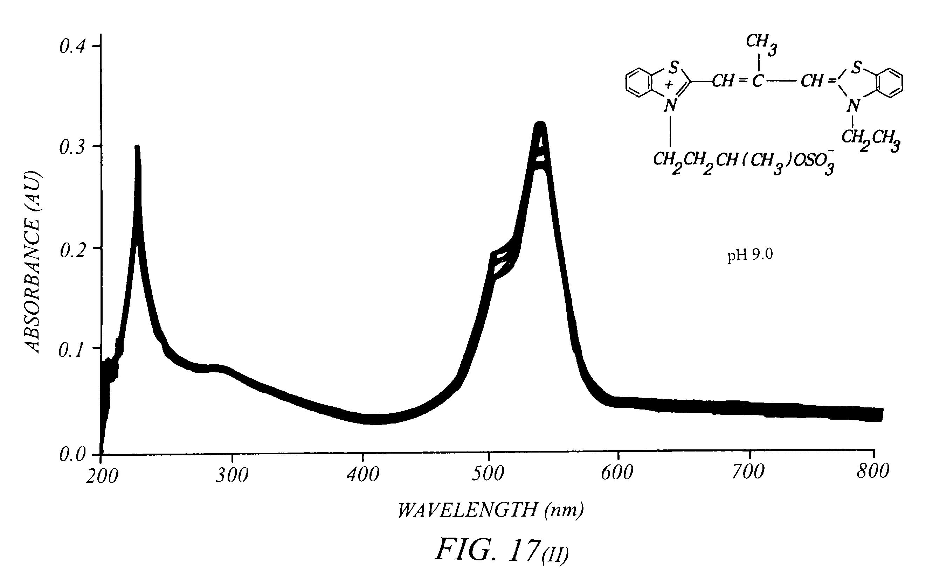

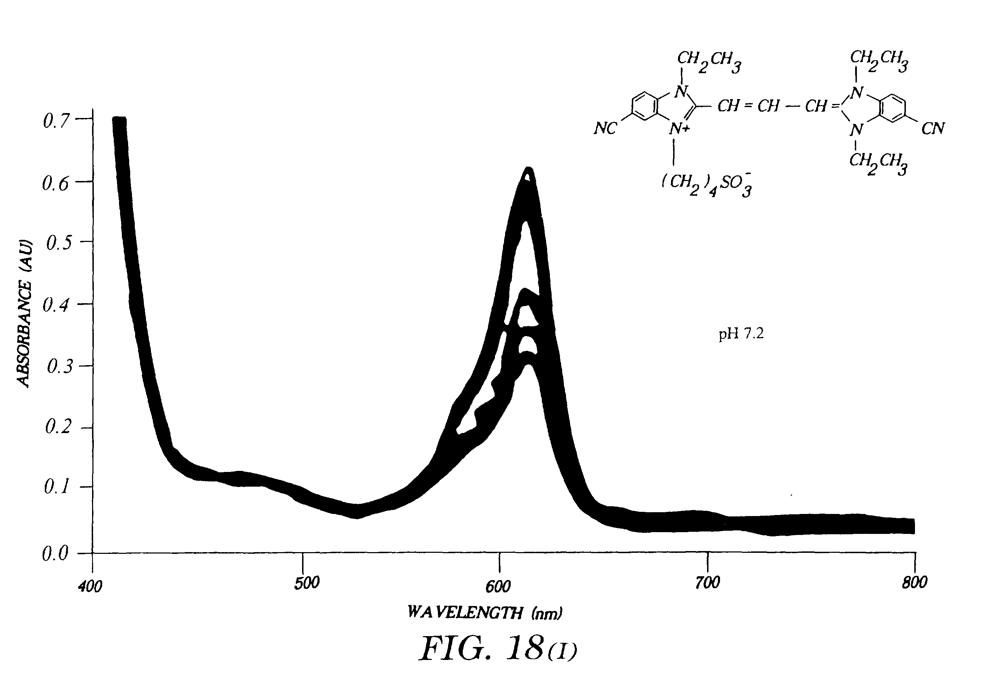

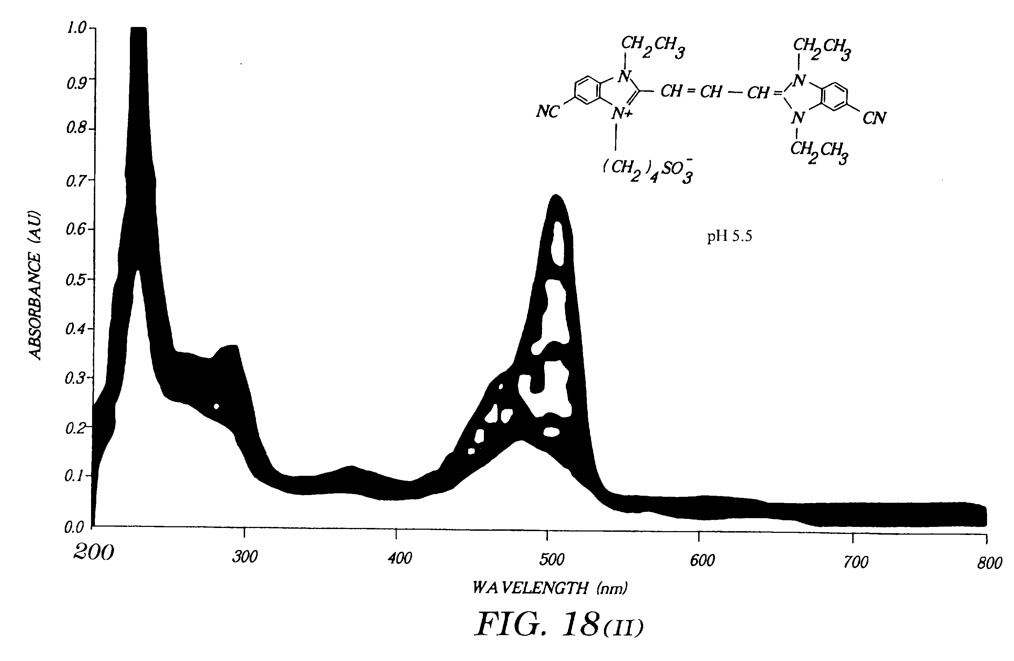

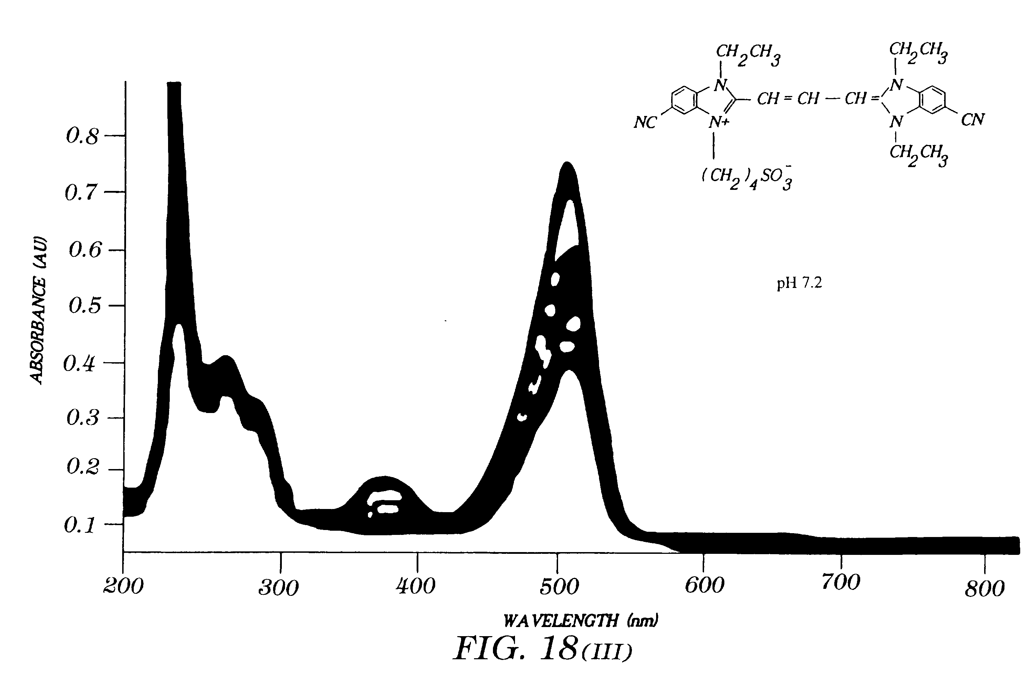

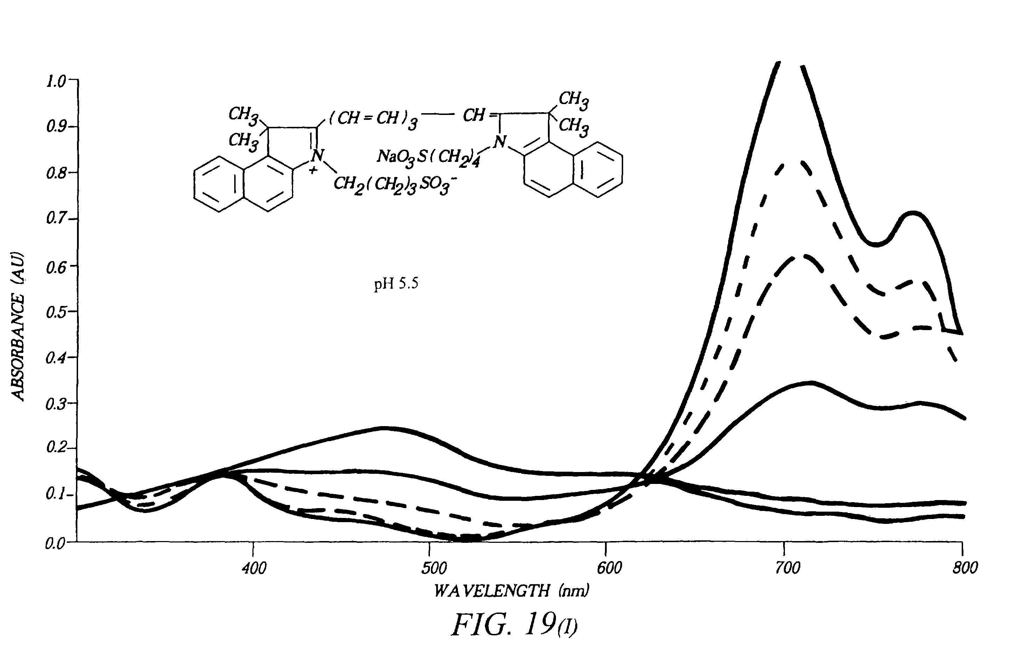

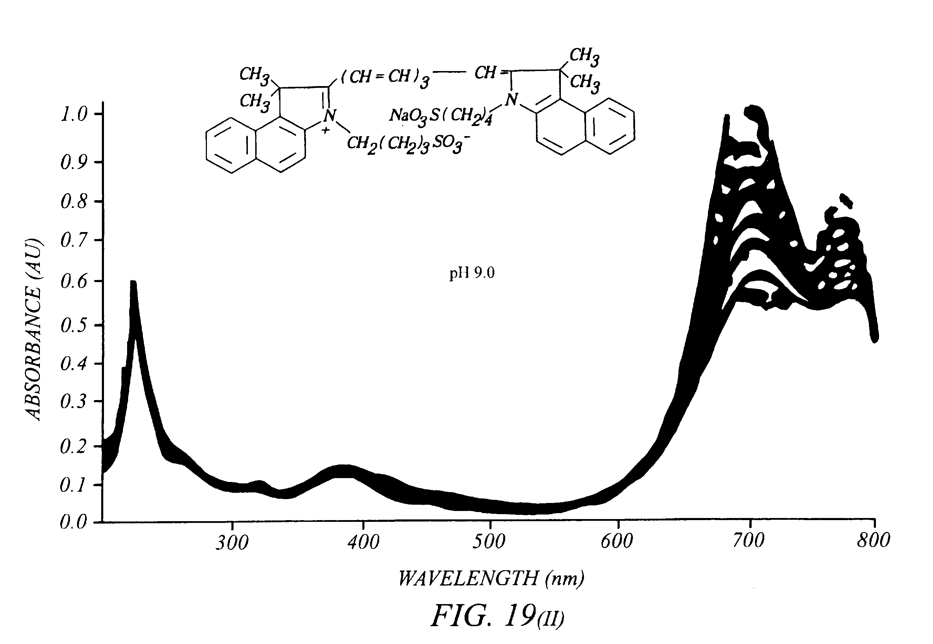

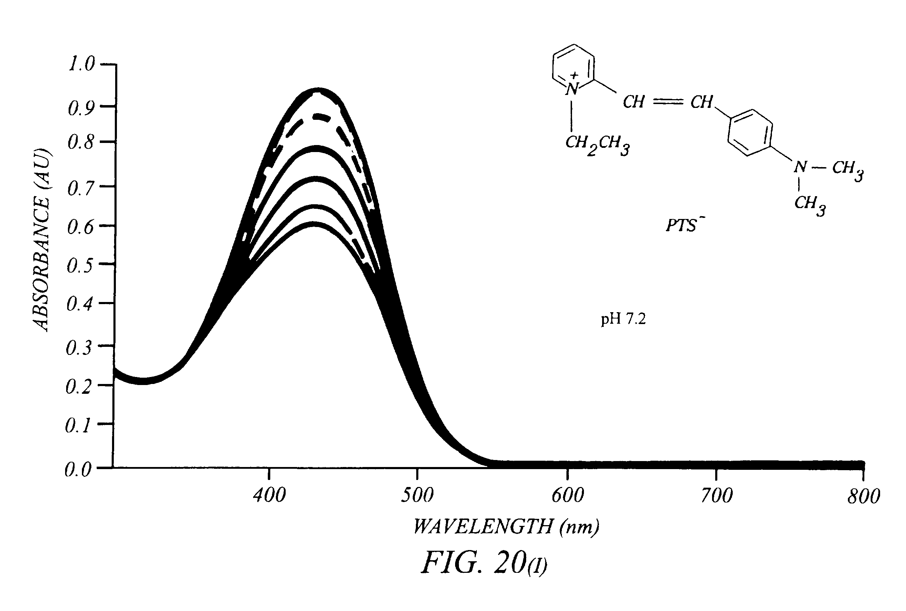

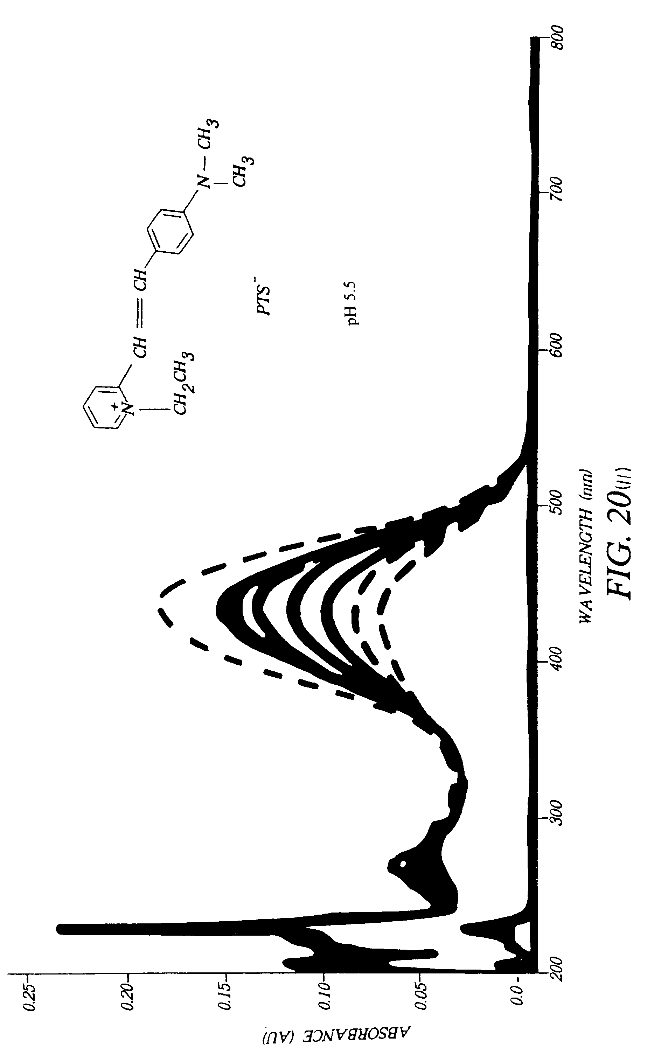

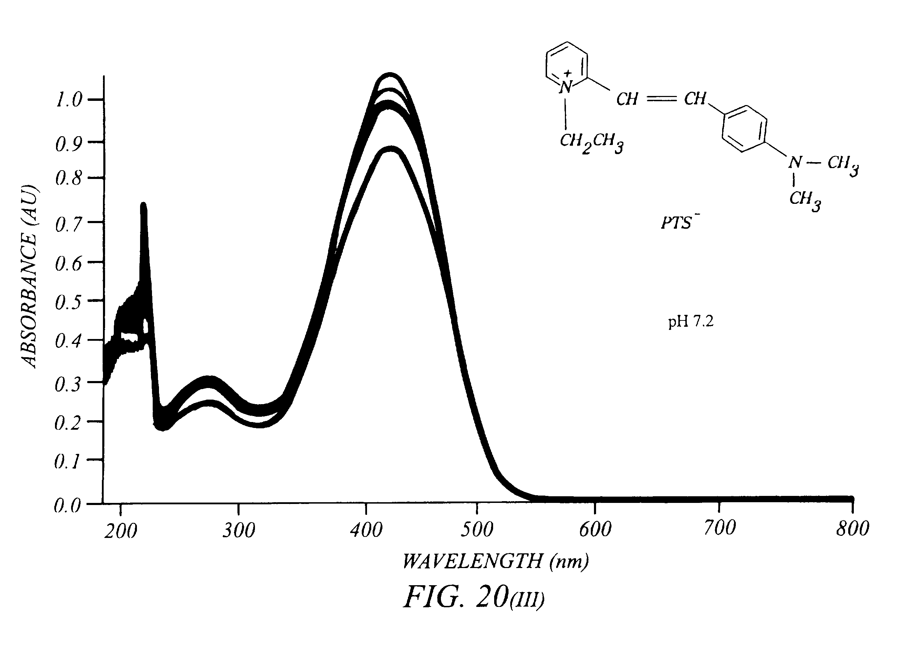

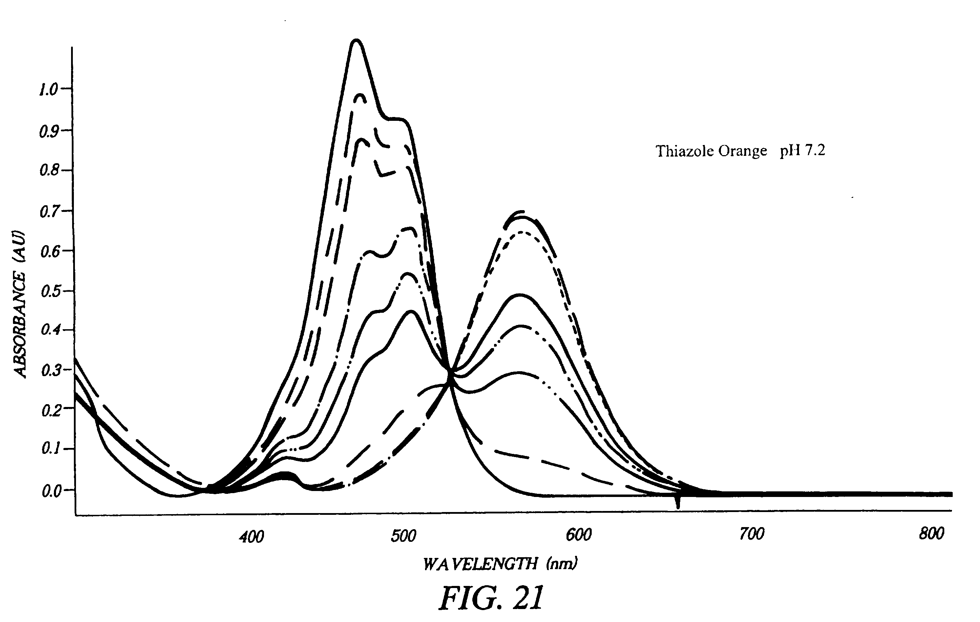

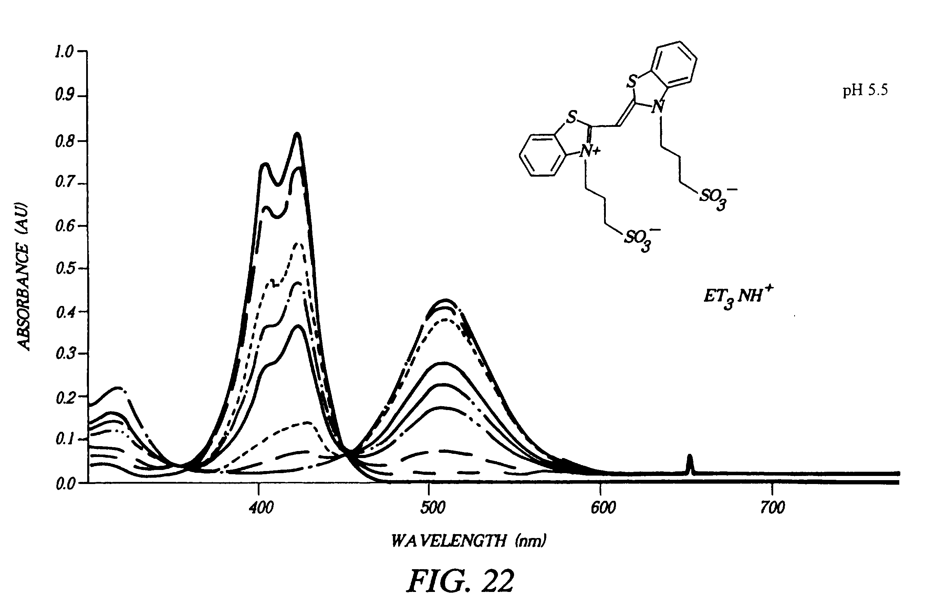

The experiments relating to Figures 12-22 were conducted as follows: the selected compound from DW3 (final concentration, 10⁻⁵-10⁻⁶ M) was dissolved in a solution of 0.05 M MES buffer (2-[N-Morpholino]ethanesulfonic acid, pH 5.5) or Tris buffer (Tris(hydroxymethyl)-aminomethane, pH 7.2) containing in addition (0.05 M) NaBr, and the initial absorption spectrum of the dye was recorded. To the sample of dye (990 uL) was then added 10 uL of a stock solution of hydrogen peroxide giving a final H₂O₂ concentration of 50 uM, and the absorption spectrum was again recorded. To the dye/H₂O₂ solution was then added 1 uL of a 0.05 mg/mL solution of vandium bromoperoxidase (giving a final enzyme concentration of 50 ng/mL) and the spectrum was recorded at one minute intervals.

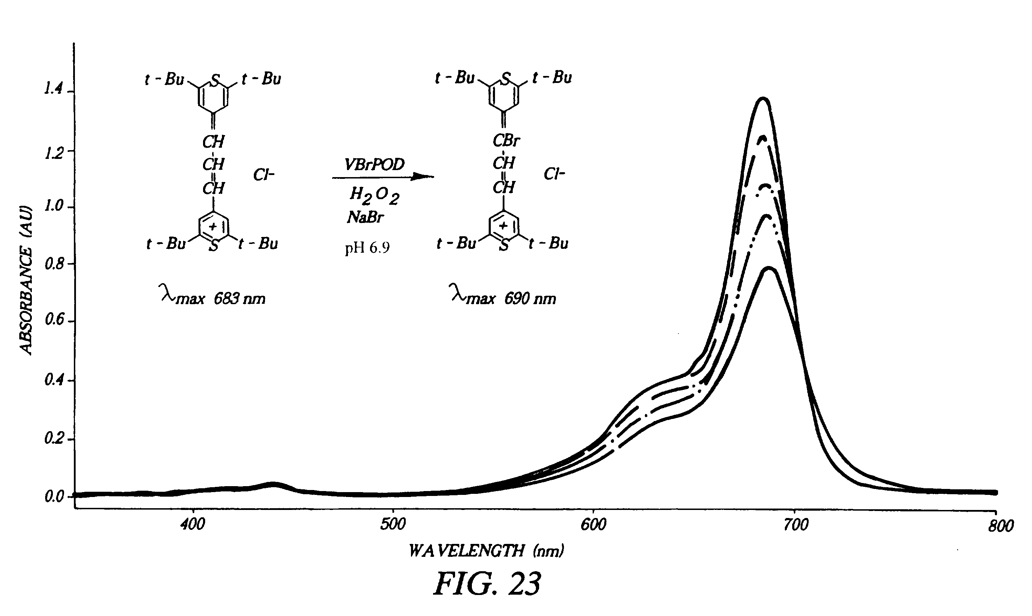

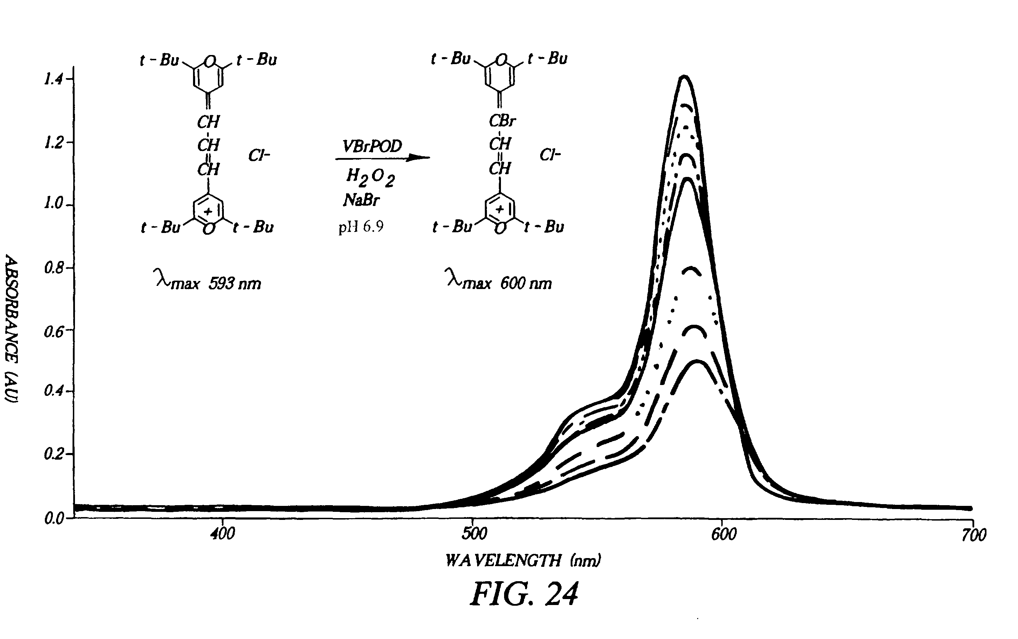

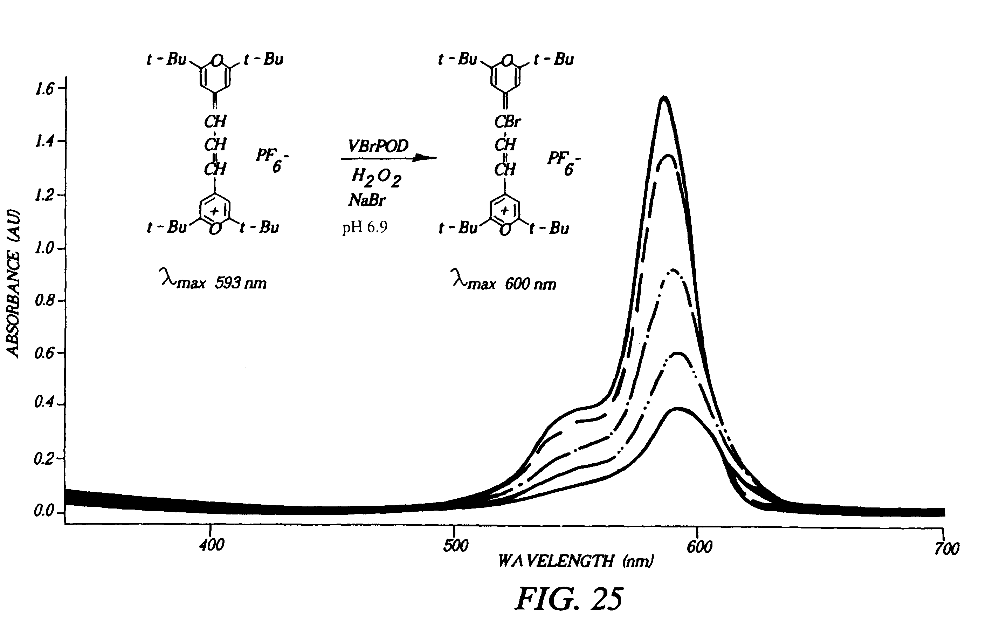

In the experiments shown in Figures 23, 24 and 25, a small volume of a 10⁻⁹ M stock solution of vanadium bromoperoxidase was added to a solution of pyrilium or thiopyrilium dye (1 x 10⁻⁵ M) in 0.05 M phosphate buffer (pH 6.9) containing 0.045 M NaBr and 2.5 x 10⁻⁴ M H₂O₂ at ambient temperature in a 1-cm pathlength quartz cuvette. Spectra were recorded on a Hewlett-Packard diode array spectrophotometer at various times after addition of enzyme. Oxidation with bromine by the enzyme resulted in a decrease of absorption at the λₘₐₓ of the starting material and an increase of absorption associated with the bromine-oxidized compound at a different wavelength. Typically, bromination of the trimethine bridge gives broadening of the absorption spectrum and isosbestic behavior on the longwavelength shoulder of λₘₐₓ. The rate of change of absorption was a function of enzyme, peroxide, and bromide concentrations.

It appears that symmetrical cyanines with multiple bridging methylenes between the two chromophores results primarily in bleaching of the dye. This observation, inter alia, may be useful, for providing multiple dye signals in a multiplexed analytical detection system.

### Halogenation of Se or Te Compounds of DW3

Depending upon the specific structure of a compound in DW3 comprising Se or Te according to the generic structural formula, it will be halogenated either at a bridging methine carbon or at the Se orTe atom.

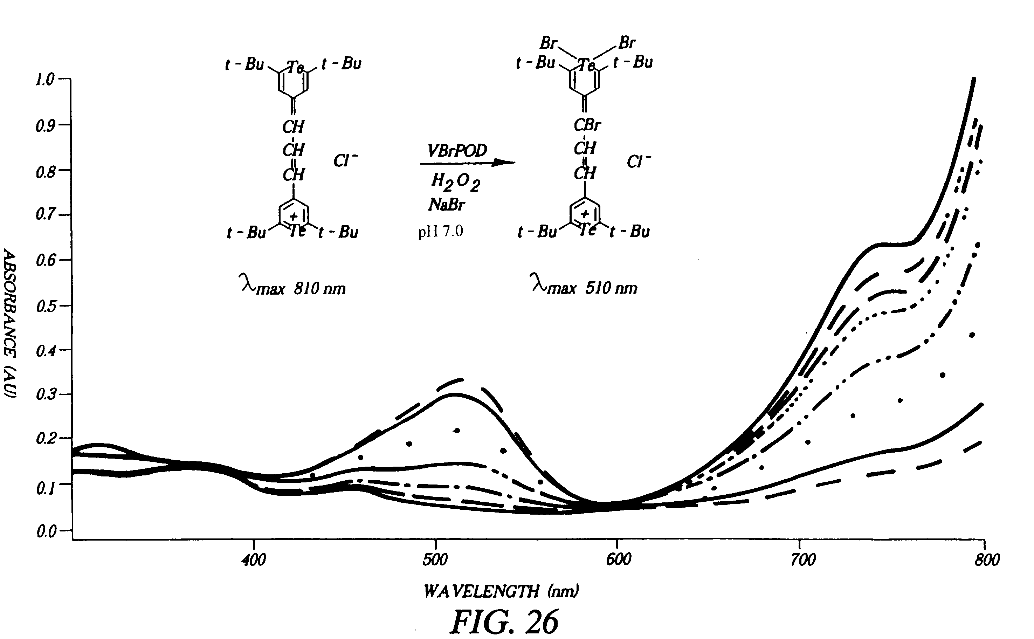

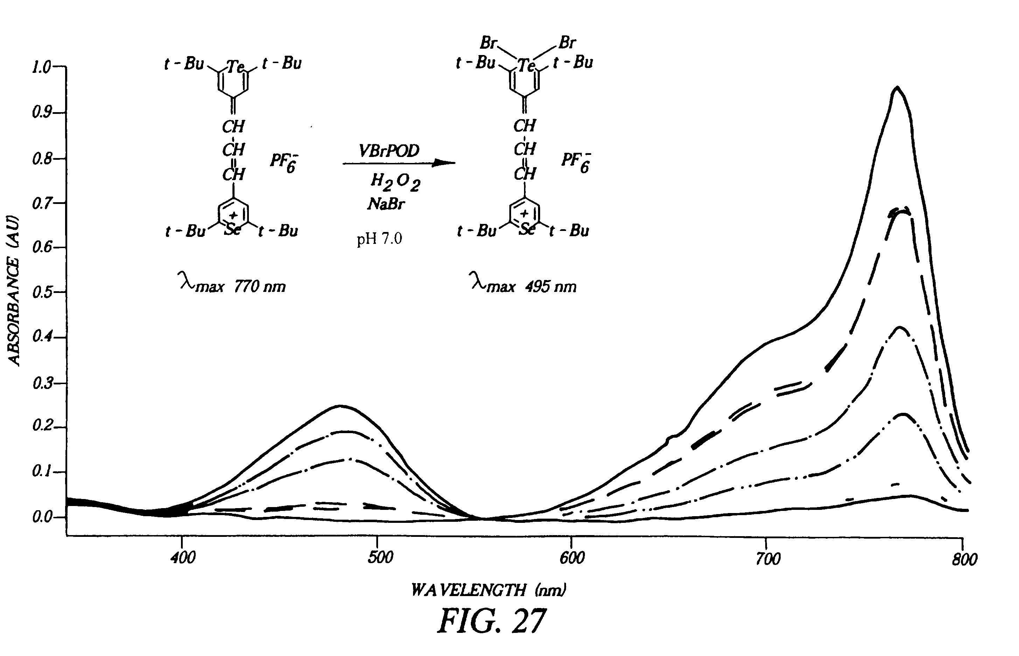

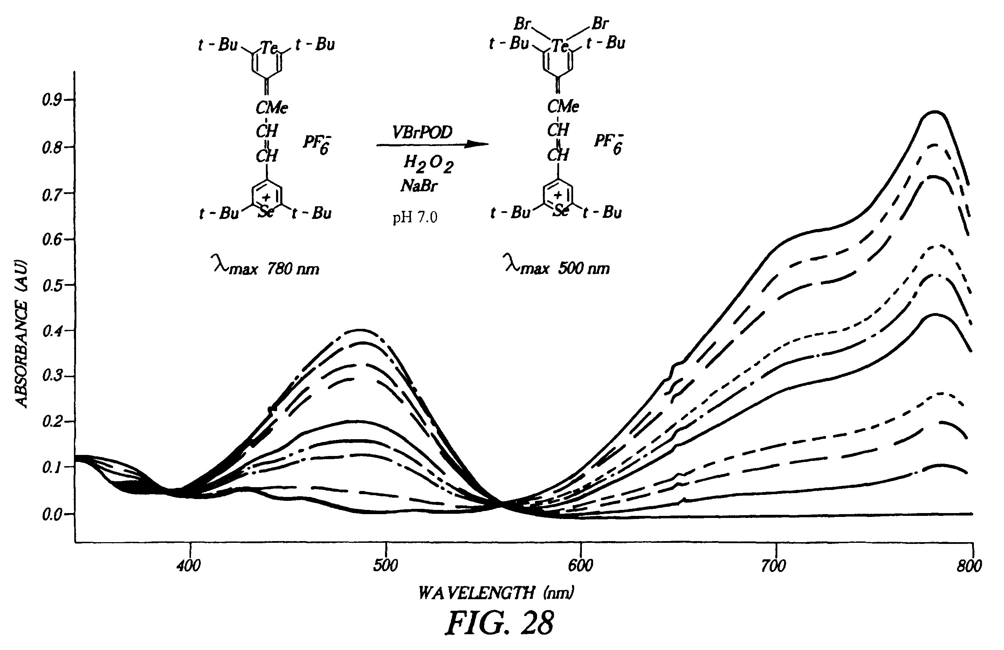

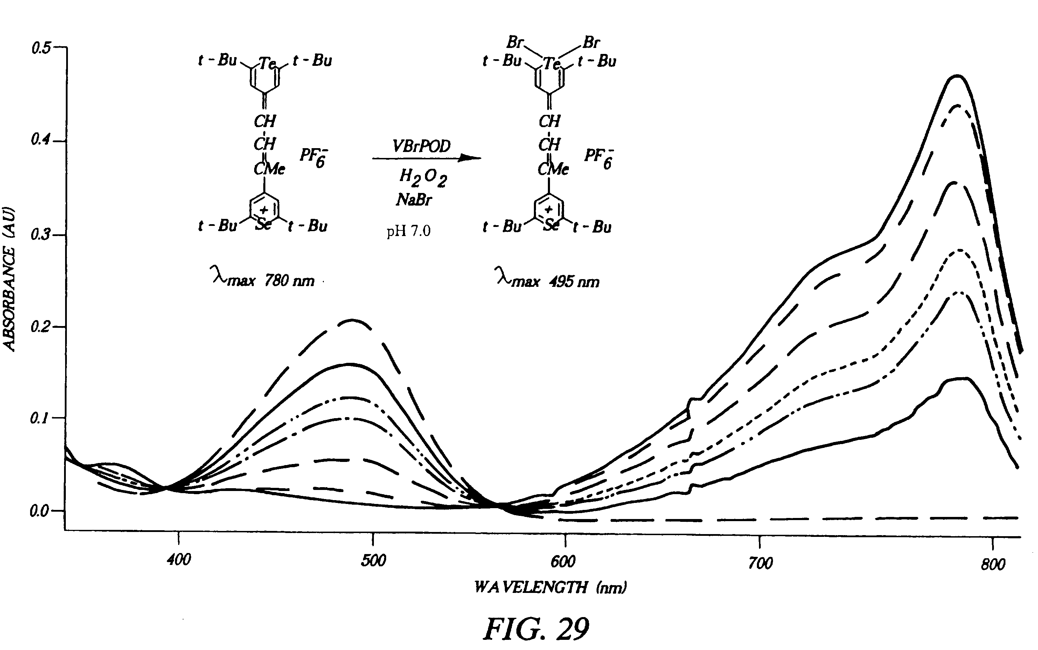

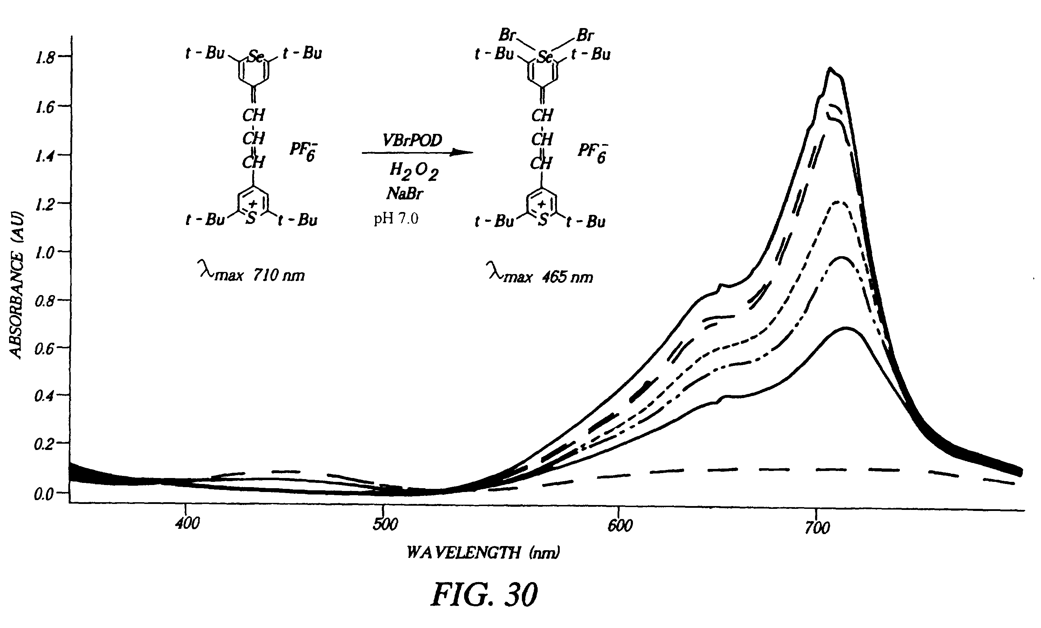

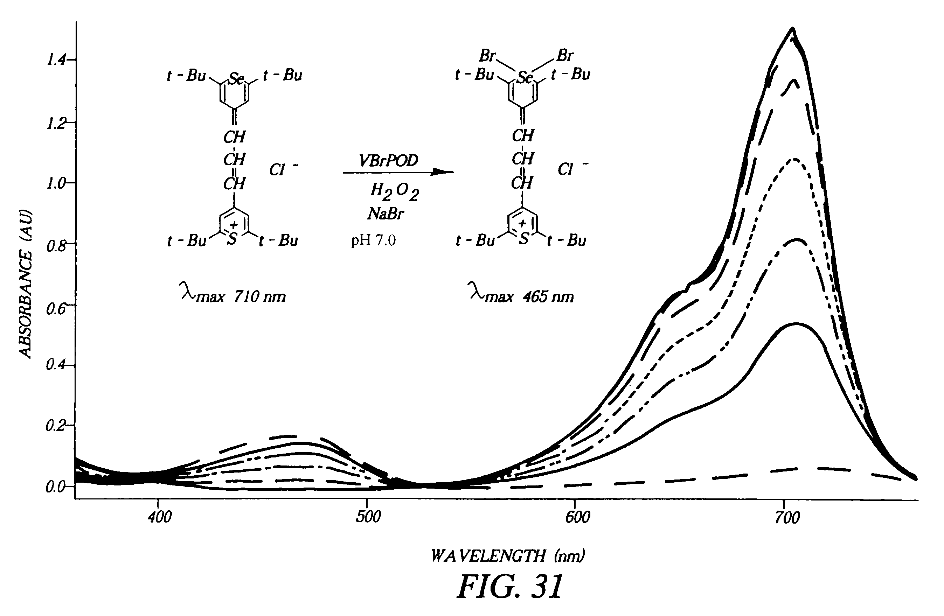

Absorption spectra are presented in Figures 26-31 for examples of compounds from DW3 that are preferentially oxidatively brominated at the Se or Te atom. The experiments were conducted as follows: vanadium bromoperoxidase (5 X 10⁻¹² - 5 X 10⁻¹¹ M ) was added to a solution of tellurium and /or selenium containing heterocycle (1 to 3 X 10⁻⁵ M) in 0.05 M phosphate buffer (pH 7) containing 0.025-0.10 M NaBr and 5 x 10⁻⁵ - 2.5 x 10⁻⁴ M H₂O₂ at ambient temperature in a 1-cm pathlength quartz cuvette. Spectra were recorded on a Hewlett-Packard diode array spectrophotometer at various times after addition of enzyme. Oxidation with bromine by the enzyme resulted in a decrease of absorption at the λₘₐₓ of the starting material and an increase of absorption at a different wavelength. The rate of change of absorption was a function of enzyme, peroxide, and bromide concentrations.

### EXAMPLE 5

### Spectral absorption changes accompanying vanadium bromoperoxidase catalyzed bromination of compounds from DW4.

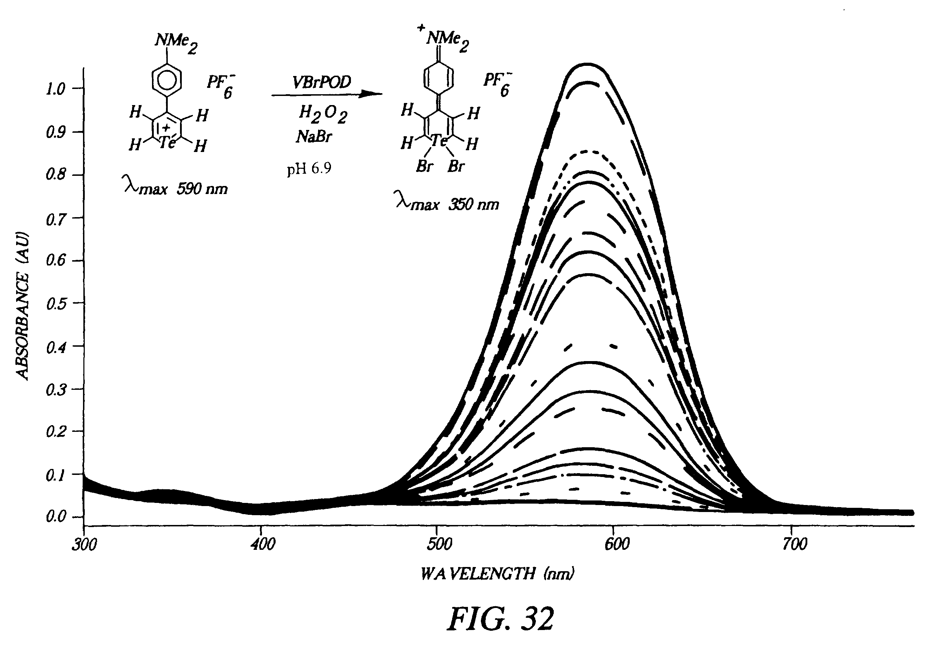

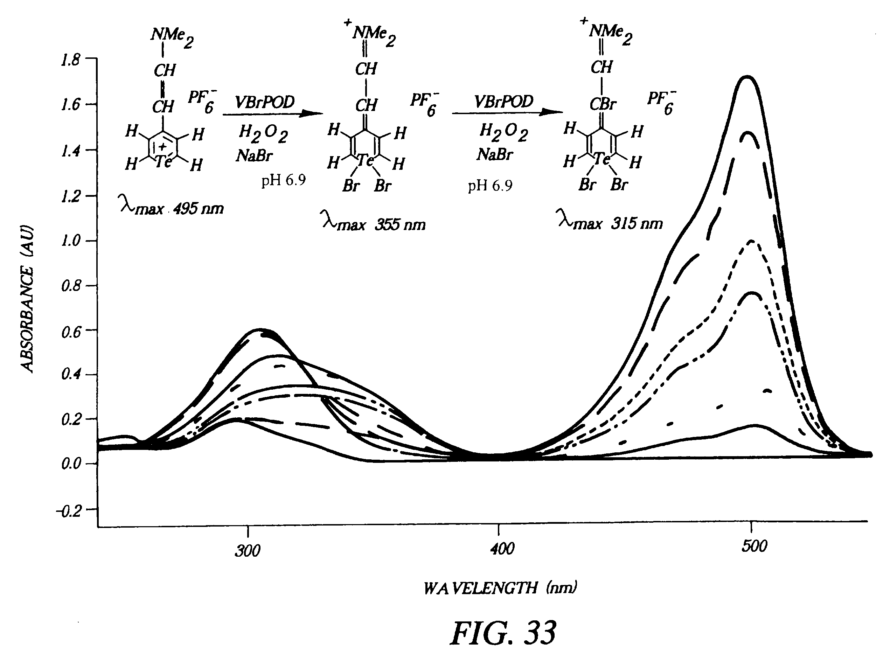

Figures 32 and 33 show the change in optical absorption accompanying bromination of two different comounds selected from DW4. The experiments were conducted as follows: in each example, 10-⁹ M stock solution of vanadium bromoperoxidase was added to a solution of tellurium and/or selenium-containing heterocycle (5 x 10⁻⁶ - 3 x 10⁻⁵ M) in 0.05 M phosphate buffer (pH 6.9) containing 0.045 M NaBr and 5 x 10⁻⁵ - 2.5 x 10⁻⁴ M H₂O₂ at ambient temperature in a 1-cm pathlength quartz cuvette. Spectra were recorded on a Hewlett-Packard diode array spectrophotometer at various times after addition of enzyme. Oxidation with bromine by the enzyme resulted in a decrease of absorption at the λmax of the starting material and an increase of absorption at a different wavelength. The rate of change of absorption was a function of enzyme, peroxide, and bromide concentrations.

### EXAMPLE 6

### Spectral absorption changes accompanying Phenol Red bromination catalyzed by vanadium bromoperoxidase.

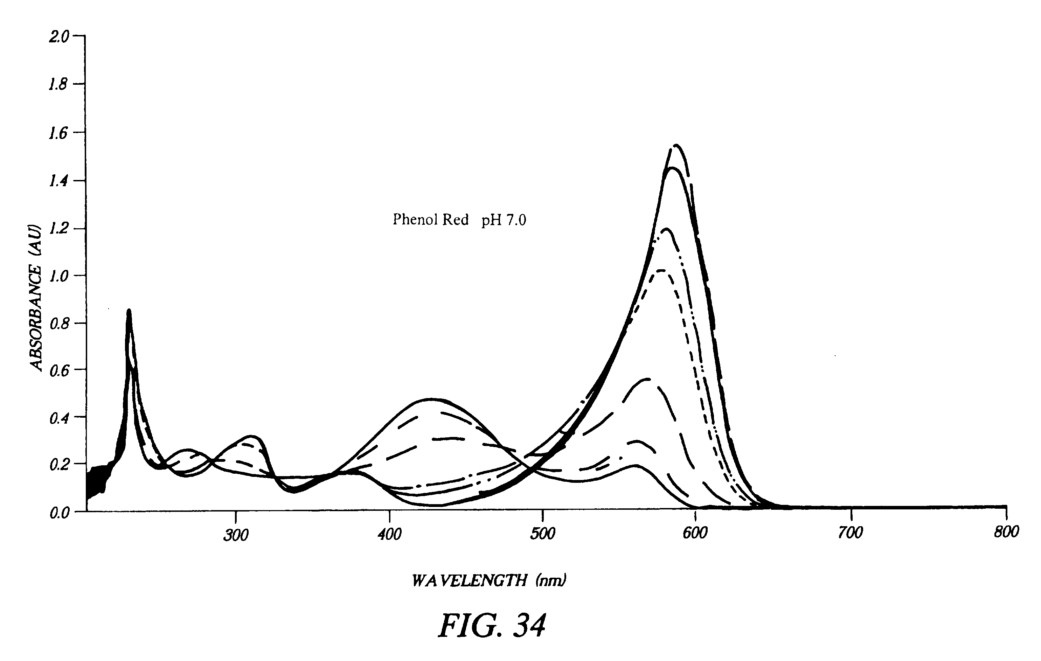

The spectral changes accompanying the bromination of phenol red, which is not a signal-generating compound of the present invention, is shown in Figure 34.

The spectral absorption change that is observed upon bromination of phenol red is due to a decrease in the pKa of the sulfonate group. As a signal-generating compound for determining the amount of an analyte, phenol red is not very useful. The absorption coefficient is small and the greatest spectral shift occurs only upon multiple bromination. Moreover, formation of a mixture of brominated products having significant concentration and spectral absorption during signal measuement is not desirable for the quantitative detection of an analyte.

### Dry Element Example

### EXAMPLE 7

### Dry element for measurement of glucose using vanadium bromoperoxidase catalyzed bromination of a signal-generating compound from DW3

A dry analytical element for the measurement of glucose was prepared as described below.

### Reagent Zone

A reagent zone was formed using a rectangular section of a polysulfone (PS) membrane (approximate dimensions 0.2mm X 7mm X 10mm) having a 0.35 micron pore size. The membrane was dipped quickly into solution A, described below, and then dried at 56° C for 10 minutes. The dried membrane then was dipped quickly into solution B, described below, and dried as before.

### Solution A

Glucose Oxidase, 500 U; VBrPOD, 536 U; Mannitol, 20 mg; Poly(methyl vinyl ether/maleic anhydride surfactant,12 mg; Hydrolyzed Collagen, 29 mg; KBr, 12 mg; 2-(4-morpholino)ethanesulfonic acid buffer, 42 mg; H₂O to final volume of 2 mL, pH 6.3.

### Solution B

1 mL 70% ethanol; 33.6 mg of the DW3 compound of Preparation 1.

### Support

A non-porous plastic strip, of approximate dimensions 1 mm X 7mm X 50mm, having a lengthwise off-center elliptical opening therethrough (minor and major diameters of about 4mm and 5mm) served as a support. The dry PS membrane comprising the components of solutions A and B was deposed on one side of the support over and symmetrically covering the elliptical opening. Thus the PS membrane comprising the reagent zone is visible through the elliptical opening through the plastic support.

### Spreading Zone

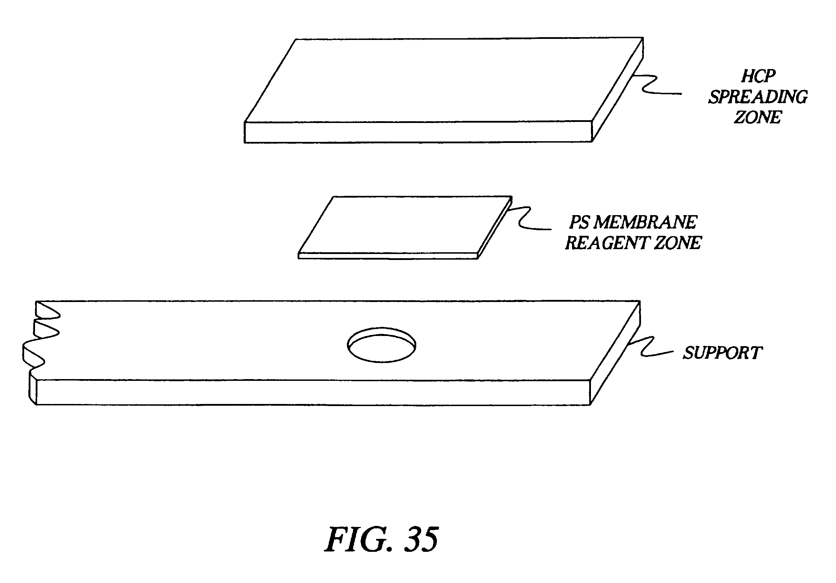

A porous hydrophilic coated polyethylene (HCP) strip, of approximate dimensions 0.5mm X 7mm X 25mm, served as the spreading zone. The HCP strip was symmetrically deposed over the PS membrane firmly, to ensure that the HCP strip and PS membrane made secure physical and therefore, effective fluid contact. The HCP strip then was glued to the support. A diagram of the dry element construction is shown in Figure 35.

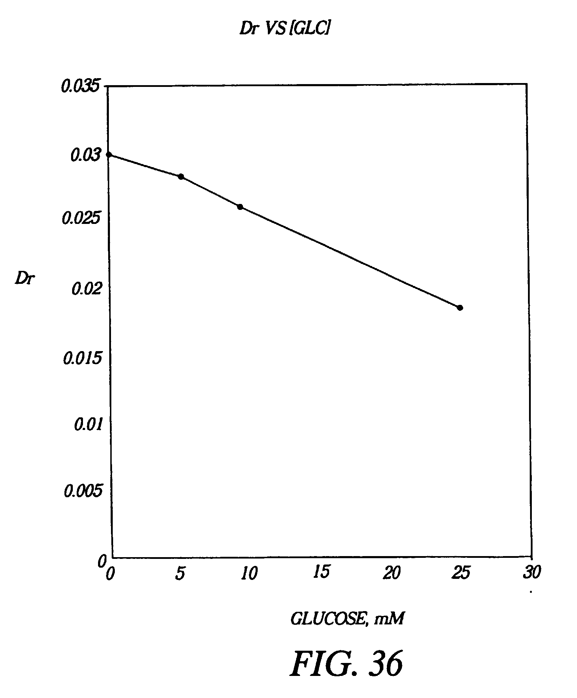

Aqueous solutions of glucose were prepared and the HCP spreading zone of individual dry analytical elements was wetted with an aliquot of each solution. The intensity of light (595 nm) transmitted through the spreading zone, reagent zone and the elliptical opening of the support was measured using a densitometer. The transmitted intensity, Dr, plotted against the glucose concentration of the applied sample is shown in Figure 36. The transmitted intensity is inversely proportional to the amount of glucose in the sample; the amount of dye formed and the amount of incident radiation absorbed by the dye increased proportionately with glucose concentration. Similar results were obtained with aqueous solutions comprising glucose and protein and glucose in whole blood.

The present invention, inter alia, provides i) analytical systems wherein competitive registration by hydrogen peroxide is much slower than registration by halogen and/or hypohalous acid, ii) spectral absorption changes that can be monitored by both rate of disappearance of reduced material and by rate of appearance of oxidized material, iii) signals and signal rates of change that are proportional to enzyme, peroxide, or halide concentration, and iv) stable signal changes.

The entire contents of all the above-cited references are herein incorporated by reference .

The invention has been described in detail with particular reference to preferred embodiments. From the examples and teachings provided herein, other aspects, advantages and embodiments of the invention will be apparent to the skilled artisan. It will be understood that variations and modifications can be effected which are within the spirit and scope of the invention.

## Claims

1. A composition comprising :
i) a haloperoxidase or haloperoxidase-labeled reactant, or a halide salt or pseudohalogen; or
ii) a haloperoxidase or haloperoxidase-labeled reactant, and a halide salt or pseudohalogen;
iii) a compound of formula 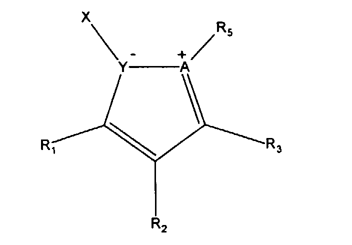 wherein Y is Te or Se; A is N, O, or S; X is Cl, Br, I, F, CN, SCN or CF₃CO₂ ; R_{1,} R₂ and R₃ are independently H, substituted or unsubstituted straight chain or branched alkyl, oxyalkyl, thioalkyl, aminoalkyl of 1 to 6 carbon atoms, substituted or unsubstituted alkenyl of 2 to 6 carbon atoms, substituted or unsubstituted aryl or heteroaryl of 6 to 14 carbon atoms, Cl, Br, I, or F, or R₁ and R₂ together form a substituted, or unsubstituted benzene ring or naphthalene ring; R₅ is an electron pair when when A is S,O; R₅ is H, substituted or unsubstituted straight chain or branched alkyl, oxyalkyl, thioalkyl, aminoalkyl of 1 to 6 carbon atoms, or substituted or unsubstituted alkenyl of 2 to 6 carbon atoms when A is N; and optionally
iv) a compound of formula 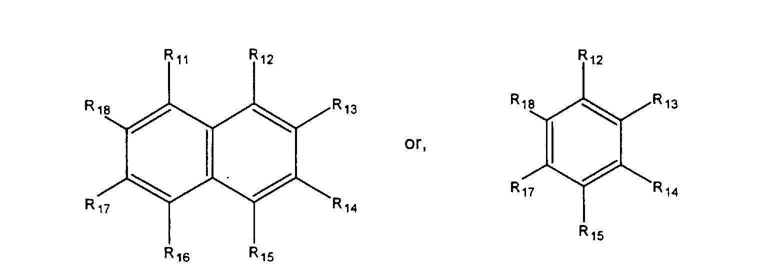 wherein R₁₁, R₁₂, R₁₃ are independently H, OH, or NMe₂ and R₁₄, R₁₅, R₁₆ are independently H, OH, NMe₂ or halogen.

2. A composition comprising :
i) a haloperoxidase or haloperoxidase-labeled reactant, or a halide salt or pseudohalogen; or
ii) a haloperoxidase or haloperoxidase-labeled reactant, and a halide salt or pseudohalogen;
iii) a compound of formula 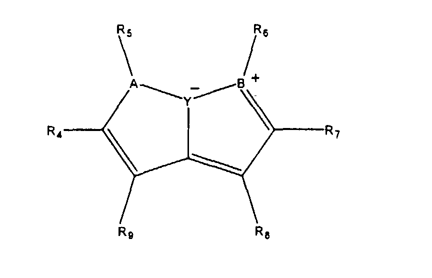 wherein Y, A, and R₅ are as defined in claim 1; B is N, O, or S; R₄ and R₇ are independently H, amine, hydroxy, substituted or unsubstituted straight chain or branched alkyl, oxyalkyl, thioalkyl, aminoalkyl of 1 to 6 carbon atoms, substituted or unsubstituted alkenyl of 2 to 6 carbon atoms, substituted or unsubstituted heterocyclic ring of 5 or 6 atoms, substituted or unsubstituted aryl or heteroaryl of 6 to 14 carbon atoms; R₈ and R₉ are independently H, amine, hydroxy, Cl, Br, I, F, substituted or unsubstituted straight chain or branched alkyl, oxyalkyl, thioalkyl, aminoalkyl of 1 to 6 carbon atoms, substituted or unsubstituted alkenyl of 2 to 6 carbon atoms, substituted or unsubstituted heterocyclic ring of 5 or 6 atoms, substituted or unsubstituted aryl or heteroaryl of 6 to 14 carbon atoms; R₄ and R₉ together form a substituted or unsubstituted benzene or naphthalene ring; R₇ and R₈ together form a substituted or unsubstituted benzene or naphthalene ring; R₆ is an electron pair when when B is S, or O; R₆ is H, substituted or unsubstituted straight chain or branched alkyl, oxyalkyl, thioalkyl, aminoalkyl of 1 to 6 carbon atoms, or substituted or unsubstituted alkenyl of 2 to 6 carbon atoms when B is N.

3. A composition comprising :
i) a haloperoxidase or haloperoxidase-labeled reactant, or a halide salt or pseudohalogen; or
ii) a haloperoxidase or haloperoxidase-labeled reactant, and a halide salt or pseudohalogen;
iii) a compound of formula 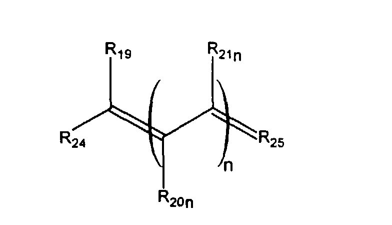 n is an integer from 0 to 8, R₁₉, R₂₀ₙ and R₂₁ₙ are independently hydrogen, halogen, cyano, amino, substituted or unsubstituted alkyl, thioalkyl, oxyalkyl, aminoalkyl, amidoalkyl or N-substituted amidoalkyl of 1 to 6 carbon atoms, substituted or unsubstituted aryl, thioaryl or oxyaryl,
R₂₄ is 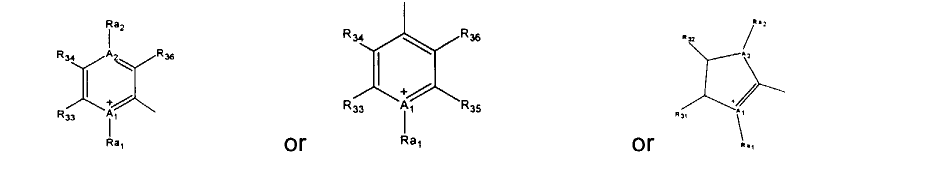 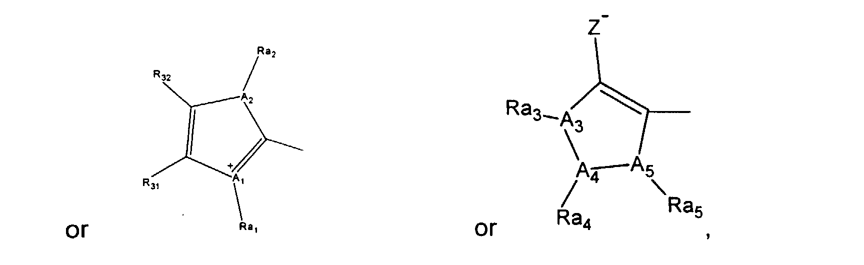 R₂₅ is 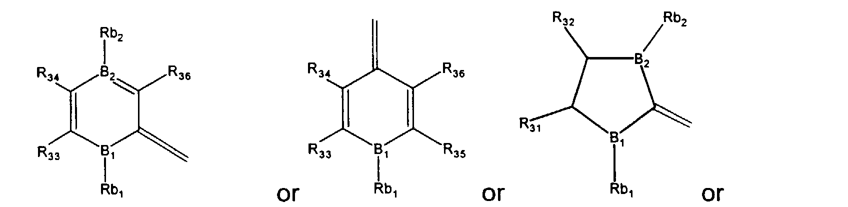 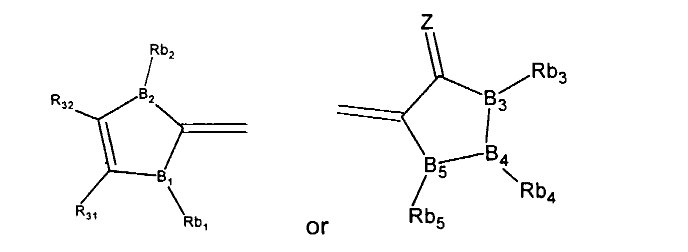 A₁,A₂,B₁ and B₂ are independently Se,Te,C,N,S,O; Ra₁, Ra₂, Rb₁ and Rb₂ are independently H,substituted or unsubstituted straight chain or branched alkyl, oxyalkyl, thioalkyl, aminoalkyl of 1 to 6 carbon atoms, or substituted or unsubstituted alkenyl of 2 to 6 carbon atoms when A₁,A₂,B₁ or B₂ is C or N, and an electron pair when when A₁,A₂,B₁ or B₂ is Se, Te, O, or S; R₃₁ and R₃₂ are independently H, amine, hydroxy, halide, acetyl, acetylamino, substituted or unsubstituted straight chain or branched alkyl, thioalkyl, aminoalkyl, oxyalkyl of 1 to 6 carbon atoms, substituted or unsubstituted alkenyl of 2 to 6 carbon atoms, substituted or unsubstituted aryl or heteroaryl of 6 to 14 atoms, or R₃₁ and R₃₂ together form a substituted or unsubstituted benzene ring, substituted or unsubstituted heterocyclic aromatic ring, or substituted or unsubstituted naphthalene ring, R₃₃, R₃₄, R₃₅ and R₃₆ are independently H, amine, hydroxy, halide, acetyl, acetylamino, substituted or unsubstituted straight chain or branched alkyl, thioalkyl, aminoalkyl, oxyalkyl of 1 to 6 carbon atoms, substituted or unsubstituted alkenyl of 2 to 6 carbon atoms, substituted or unsubstituted aryl or heteroaryl of 6 to 14 carbon atoms, or R₃₃ and R₃₄ together form a substituted or unsubstiruted benzene ring, substituted or unsubstituted heterocyclic aromatic ring, or a substituted or unsubstituted naphthalene ring; R₃₅ and R₃₆ together form a substituted or unsubstiruted benzene ring, substituted or unsubstituted heterocyclic aromatic ring, or a substituted or unsubstituted naphthalene ring; Z is S or O, A₃, A₄, A₅, B₃, B₄, and B₅ are independently C, O, N or S, when A₃, A₄, A₅, B₃, B₄ or B₅ is C or N then Ra₃, Ra₄, Ra₅, Rb₃, Rb₄ or Rb₅ are independently H, S, O, amino, substituted or unsubstituted straight chain or branched alkyl, thioalkyl, aminoalkyl of 1 to 6 carbon atoms, substituted or unsubstituted aryl or heteroaryl, when A₃, A₄, A₅, B₃, B₄, or B₅ is O or S then Ra₃, Ra₄, Ra₅, Rb₃, Rb₄ or Rb₅ is an electron pair.

4. A composition comprising :
i) a haloperoxidase or haloperoxidase-labeled reactant, or a halide salt or pseudohalogen; or
ii) a haloperoxidase or haloperoxidase-labeled reactant, and a halide salt or pseudohalogen;
iii) a compound of formula 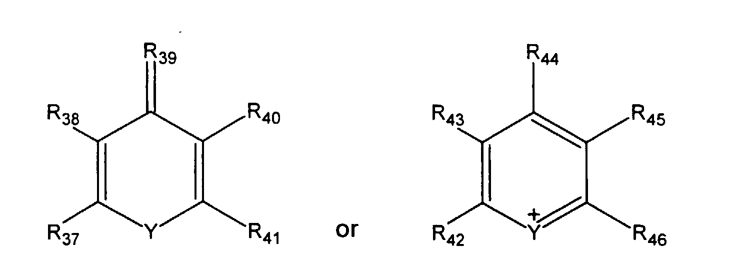 Y is Te or Se; R₃₉ is O, -C(CN)₂, R₃₇, R₃₈, R₄₀, R₄₁ are independently H, R₄₂; R₄₃, R₄₄, R₄₅, R₄₆, are independently H, R₄₄ is substitited or unsubstituted aryl, R₄₂ and R₄₃ together form a substituted or unsubstituted benzene ring, R₄₅ and R₄₆ together form a substituted or unsubstituted benzene ring.

5. A dry analytical element comprising:
i) a spreading zone;
ii) one or more reagent zones; and
iii) a support;
wherein at least one of the zones comprises :
a) a compound of formula 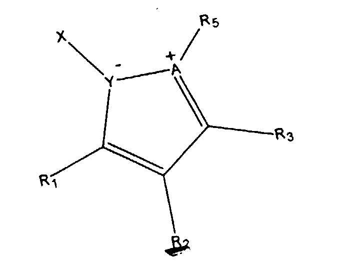 as defined in claim 1 and optionally a compound of the formula 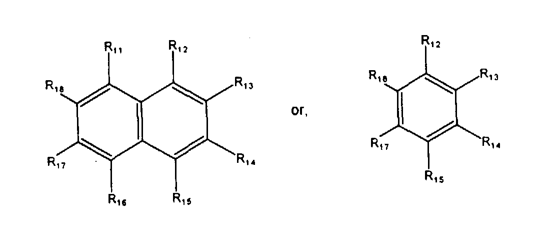 as defined in claim 1; or
b) a compound of formula 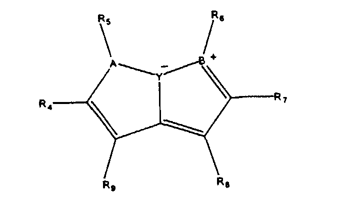 as defined in claim 2; or
c) a compound of formula 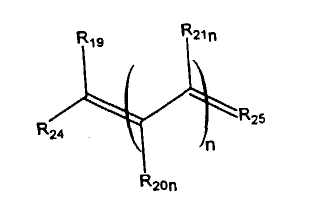 as defined in claim 3; or
d) a compound of formula 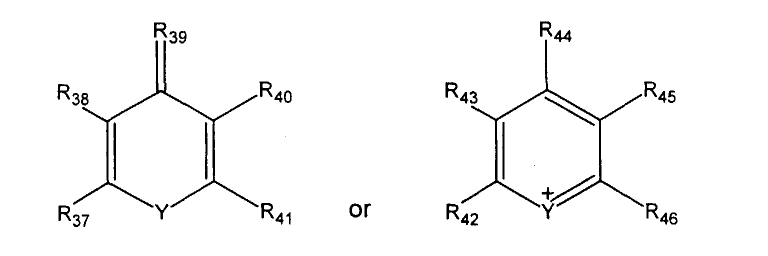 as defined in claim 4.

6. A method for determining a peroxide comprising the steps of:
a) contacting a sample suspected of containing a peroxide with
i)a) a compound of formula 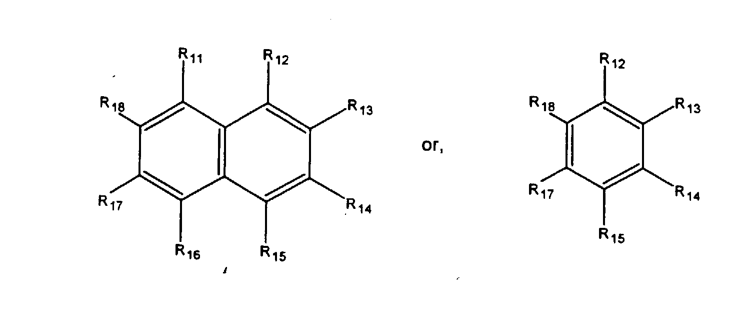 as defined in claim 1 and optionally a compound of of formula 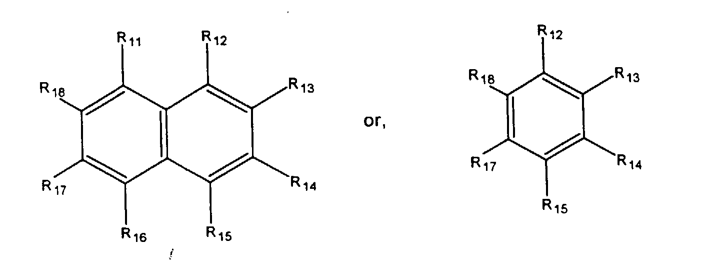 as defined in claim 1; or
i)b) a compound of formula 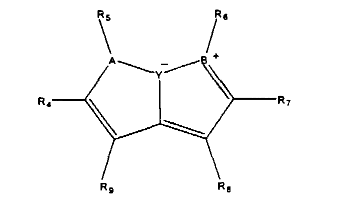 as defined in claim 2; or
i)c) a compound of formula 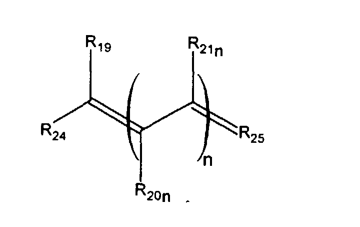 as defined in claim 3; or
i)d) a compound of formula 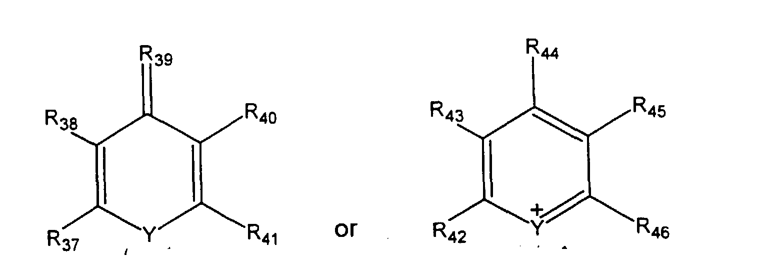 as defined in claim 4,
ii) a halide salt or pseudohalogen,
iii) a haloperoxidase, thereby producing a change in optical absorption; and
c) detecting the change in optical absorption as a measure of the amount of peroxide in the sample.

7. The method of claim 6 wherein the haloperoxidase is a vanadium bromoperoxidase, the halide salt is a bromide salt, and the peroxide is hydrogen peroxide.

8. The method of claim 7 wherein the vanadium bromoperoxidase is from Ascophylum nodosum.

9. Use of the compound(s) as defined in any one of claims 1 to 4 in an assay, such as a sandwich assay, for peroxide or a peroxide producing analyte.
